# EUROPEAN PATENT APPLICATION

(11) **EP 2 327 307 A1**
(43) Date of publication of application: **01.06.2011**
(21) Application number: 09814610.3
(22) Date of filing: 16.09.2009
(51) Int. Cl.: A01N 59/00, A01G 7/00, A01G 7/06, A01N 25/02, A01N 59/06, A01N 59/16, A01N 59/20, A01P 3/00, C02F 1/30, C02F 1/32, C02F 1/36, C02F 1/72, C02F 1/74, C02F 1/78

(54) **WATER THAT EXPRESSES PATHOGEN-RESISTANCE GENES (PR GENE CLUSTERS) TO ENCODE PLANT IMMUNOPROTEINS, A METHOD OF PREVENTING PLANT DISEASES USING THE WATER, AND A DEVICE FOR PRODUCING THE WATER**

(30) Priority: 16.09.2008 JP 2008235861
(71) Applicant: Kitakyushu Foundation for the Advancement of Industry, Science and Technology, Kitakyushu-shi Fukuoka 808-0135 (JP)
(72) Inventor: TANAKA Kenichiro, Kitakyushu-shi Fukuoka 807-0871 (JP); TANAKA Licca, Kitakyushu-shi Fukuoka 807-0871 (JP); KAWANO Tomonori, Kitakyushu-shi Fukuoka 807-0876 (JP)
(74) Representative: Uchida, Kenji
(86) International application number: PCT/JP2009/066198
(87) International publication number: WO 2010/032765

(57) **Abstract**

Disclosed is a method of preventing diseases in plants which can bring about a redox reaction in the inside of plant cells thus inducing the expression of pathogen-resistance genes in the plant cells. There is no possibility that residual contents remain in soils. It is also possible to cultivate plants which are strong against diseases. By bringing water which contains reactive oxygen species and allows the reactive oxygen species to be held and to function for a long period into contact with plants, the expression of the pathogen-resistance genes which the plants possess is induced. Accordingly, the water containing reactive oxygen is absorbed into the plants to prevent diseases in plants.

## Description

### Technical Field

The present invention relates to water that expresses pathogen-resistance genes (PR gene group) to encode plant immunity stimulating immunoproteins, a method of preventing diseases in plants using the water, and an apparatus for producing the water.

### Background Art

Conventionally, there has been known a means which makes use of an immune-protective mechanism which plants originally possess for preventing diseases in plants.

That is, the plant has the immune-protective mechanism which enhances its own resistance against the invasion of pathogens. The resistance against diseases in plants can be enhanced by allowing the plant to express an immunity stimulating material which generates such a protective mechanism.

To allow the plant to express the immunity stimulating material, for example, benzo (1,2,3) thiadiazole-7-carbothioic acid S-methyl ester (BTH) (made by E.I. du Pont de Numours and Company, USA) or probenazole (product name: Oryzemate) made by Meiji (see patent document 1) is used.

### Prior art literature

Patent Literature
Patent literature: JP-A-2006-327995

### Disclosure of the Invention

### Problems that the Invention is to solve

However, since the above-mentioned conventional method of preventing diseases in plants uses a chemical, in a farming season where a large quantity of chemical is used, the chemical dissolves into a drainage canal and rivers, and there exists a possibility that an amount of dissolved chemical exceeds a prescribed standard. Further, as an example which anticipates the increase of a load on an environment, on a data sheet of a commercially available probenazole (Hokho: lateral-row Oryzemate granular wettable powder), a warning on fish toxicity (LC50 value (48 hours) for carp: 8ppm) is called.

On the other hand, inventors of the present invention have made extensive studies and have found a phenomenon that active oxygen species induce the expression of disease-resistance genes of a plant. Accordingly, it is thought that the disease resistance of a plant can be enhanced by bringing these active oxygen species into contact with a plant.

However, in a method which brings active oxygen species into contact with a plant in a gaseous form or in a mist form, since active oxygen species is brought into contact with air, active oxygen species acts on organic substances or floating bacteria in the atmosphere and is consumed. Accordingly, the concentration of active oxygen species which reaches a plant body on which active oxygen species acts takes a low value and hence, active oxygen species of extremely high initial concentration becomes necessary for expression of the action whereby there arises a possibility of influence exerted on environment in the same manner as the above-mentioned chemical.

In view of the above, it is thought to be ideal to bring a state where active oxygen species is impregnated into water, to maintain active oxygen species in water as long as possible, and to bring the water into contact with a plant.

However, a method which constantly produces water containing such active oxygen species (hereinafter referred to as "active oxygen containing water") has not been established up to now, and the use of such water for the enhancement of disease resistance of a plant has not been studied.

The present invention has been made under such circumstances, and it is an object of the present invention to provide water containing active oxygen species which can induce the expression of pathogen-resistance genes by inducing a redox reaction in plant cells without using a chemical as an effective content, a method of preventing diseases in plants using the water, and an apparatus for producing the water.

### Means for solving the problem

To overcome the above-mentioned drawbacks of the related art, according to the present invention called for in Claim 1, there is provided active oxygen containing water containing first active oxygen species and second active oxygen species which are selected from a group consisting of superoxide anion radicals, hydroxy radicals, singlet oxygen and oxygen-containing organic radical species and differ from each other in behavior with time, and one of the first active oxygen species and the second active oxygen species exhibits lower reactivity and is capable of keeping a function thereof for a longer time compared to the other active oxygen species so that oxidizing and reducing power which the active oxygen species possess is made to reach the inside of the plant cells from the outside of the plant cells and brings about a redox reaction in the inside of the plant cells thus inducing the expression of pathogen-resistance genes in the plant cells.

Further, according to the invention called for in claim 2, there is provided a method of preventing diseases in plants in which diseases in plants are prevented by bringing water containing active oxygen species and is capable of keeping a function of the active oxygen species for a long period into contact with plants so that the expression of pathogen-resistance genes which the plants possess is induced whereby the expression of the systematic acquired resistance is induced thus preventing diseases of the plant.

Further, according to the invention called for in claim 3, in the method of preventing diseases in plants called for in claim 2, the active oxygen containing water contains at least one active oxygen which is selected from a group consisting of superoxide anion radicals, hydroxy radicals, singlet oxygen and oxygen-containing organic radicals species so that oxidizing and reducing power which the active oxygen species possess is made to reach the inside of the plant cells from the outside of the plant cells by means of a plant contact means, and the expression of pathogen-resistance genes is induced due to a redox reaction which is brought about in the inside of the plant cells by the absorbed water whereby the plant diseases are prevented.

Further, according to the invention called for in claim 4, in the method of preventing diseases in plants called for in claim 2, the active oxygen containing water contains first active oxygen species and second active oxygen species which are selected from a group consisting of superoxide anion radicals, hydroxy radicals, singlet oxygen and oxygen-containing organic radical species and differ from each other in behavior with time, and one of the first active oxygen species and the second active oxygen species exhibits lower reactivity and is capable of keeping a function thereof for a longer time compared to the other active oxygen species, and in inducing the pathogen-resistance genes, the first active oxygen species which is capable of directly acting on pathogenic bacteria is made fast-acting and, subsequently, the second active oxygen species which is capable of inducing the expression of the pathogen-resistance in the plant is made slow-acting thus preventing diseases in plants.

Further, according to the invention called for in claim 5, in the method of preventing diseases in plants called for in any one of claim 2 to 4, the active oxygen species is produced by applying at least one of ultraviolet rays, ultrasonic oscillations, a visible light, microwaves to a catalytic body immersed into water.

Further, according to the invention called for in claim 6, in the method of preventing diseases in plants called for in claim 5, the water is water in which at least one of an oxygen gas, an ozone gas, a chloride gas, a nitrogen monoxide gas and an ammonium gas which constitutes a precursor of the active oxygen species is dissolved.

Further, according to the invention called for in claim 7, in the method of preventing diseases in plants called for in claim 5 or 6, the catalytic body contains at least one selected from a group of metals constituted of metal oxide ions or metal hydroxide ions of titania (TiO₂), alumina(Al₂O₃), anodized aluminum, magnesium oxide, magnesium hydroxide, magnetite (Fe₃O₄), zinc oxide, tungsten oxide, barium titanate, strontium titanate, sodium titanate, zirconium dioxide, tungsten oxide, a tungsten hydroxide compound, α- Fe₂O₃, cadmium sulfide, zinc sulfide, platinum, copper, palladium.

Further, according to the invention called for in claim 8, in the method of preventing diseases in plants called for in claims 5 to 7, the catalytic body has a powdery form, a granular form or a fibrous form.

Further, according to the invention called for in claim 9, in the method of preventing diseases in plants called for in claims 2 to 7, the active oxygen containing water is produced by an active oxygen containing water producing apparatus in which an ultraviolet-ray light source is arranged in water and a periphery of the ultraviolet-ray light source is surrounded by a photocatalytic body formed of a fibrous body with a fixed gap held between the ultraviolet-ray light source and the photocatalytic body whereby a water flow is formed of a first water flow which ascends and descends by passing the gap defined between the ultraviolet-ray light source and the photocatalytic body, a second water flow which ascends and descends by spirally passing on an outer periphery of the photocatalytic body, and a third water flow which flows into or flows out from the texture of the photocatalytic body.

Further, according to the invention called for in claim 10, there is provided an active oxygen containing water producing apparatus used in the method of preventing diseases in plants according to any one of claims 2 to 9, wherein the apparatus includes: an ultraviolet-ray light source which is arranged in a vessel provided with a water supply port and an active oxygen containing water takeout port; and a photocatalytic body which is formed of a fibrous body and surrounds a periphery of the ultraviolet-ray light source with a predetermined gap held between the photocatalytic body and the ultraviolet-ray light source, wherein a water flow supplied to the inside of the vessels is formed of a first water flow which ascends and descends by passing the gap defined between the ultraviolet-ray light source and the photocatalytic body, a second water flow which ascends and descends by spirally passing on an outer periphery of the photocatalytic body, and a third water flow which flows into or flows out from the texture of the photocatalytic body.

Further, according to the invention called for in claim 11, there is provided an active oxygen containing water producing apparatus used in the method of preventing diseases in plants according to any one of claims 2 to 9, wherein the apparatus includes: a vessel which is made of a material which allows the transmission of ultraviolet rays therethrough, is provided with a water supply port and a water discharge port and houses a catalytic body in the inside thereof; an ultrasonic irradiation part which irradiates ultrasonic waves in the flow direction of water which flows into the inside of the vessel flows from the water supply port; and an ultraviolet-ray irradiation part which is arranged on the periphery of the vessel and irradiates ultraviolet rays to the catalytic body from the outside of the vessel, wherein at least a portion of the catalytic body is arranged in an area where an irradiation area to which ultrasonic waves are irradiated from the ultrasonic irradiation part and an irradiation area to which ultraviolet rays are irradiated from the ultraviolet-ray irradiation part overlap with each other.

Further, according to the invention called for in claim 12, the active oxygen containing water producing apparatus further includes a microwave irradiation part which, when water which flows into the vessel from the water supply port flows toward the water discharge port, irradiates microwaves in the direction orthogonal to the direction along which the water flows in the inside of the vessel, wherein at least a portion of the catalytic body is arranged in an area where an irradiation area to which microwaves are irradiated from the microwave irradiation part, an irradiation area to which ultrasonic waves are irradiated from the ultrasonic irradiation part and an irradiation area to which ultraviolet rays are irradiated from the ultraviolet-ray irradiation part overlap with each other.

### Advantage of the Invention

According to the invention called for in claim 1, the active oxygen containing water contains the first active oxygen species and the second active oxygen species which are selected from a group consisting of superoxide anion radicals, hydroxy radicals, singlet oxygen and oxygen-containing organic radical species and differ from each other in behavior with time, and one of the first active oxygen species and the second active oxygen species exhibits lower reactivity and is capable of keeping a function thereof for a longer time compared to the other active oxygen species so that oxidizing and reducing power which the active oxygen species possess is made to reach the inside of the plant cells from the outside of the plant cells and brings about a redox reaction in the inside of the plant cells thus inducing the expression of pathogen-resistance genes in the plant cells whereby the active oxygen containing water becomes active oxygen containing water which can induce the expression of pathogen-resistance genes . Accordingly, it is possible to provide water containing active oxygen species which can induce the expression of pathogen-resistance genes by inducing a redox reaction in plant cells without using a chemical as an effective content.

Further, according to the method of preventing diseases in plants called for in claim 2, plant diseases are prevented by bringing water containing active oxygen species and is capable of keeping a function of the active oxygen species for a long period into contact with plants so that the expression of pathogen-resistance genes which the plants possess is induced whereby the systemic acquired resistance is expressed. Accordingly, by allowing the plant to absorb the water containing active oxygen species, a redox reaction is induced in the plant cells so that the expression of pathogen-resistance genes can be induced. Further, there is no possibility that residual contents remain in soils, and it is also possible to cultivate plants which are strong against diseases. That is, it is possible to provide water containing active oxygen species which can induce the expression of pathogen-resistance genes by inducing a redox reaction in plant cells without using a chemical as an effective content.

Further, according to the method of preventing diseases in plants called for in claim 3, the active oxygen containing water contains at least one active oxygen which is selected from a group consisting of superoxide anion radicals, hydroxy radicals, singlet oxygen and oxygen-containing organic radicals so that oxidizing and reducing power which the active oxygen species possess is made to reach the inside of the plant cells from the outside of the plant cells by means of a plant contact means, and the expression of pathogen-resistance genes is induced due to a redox reaction which is brought about in the inside of the plant cells by the absorbed water. Accordingly, it is possible to cultivate plants which are strong against diseases in various cultivation modes.

Further, according to the method of preventing diseases in plants called for in claim 4, the above-mentioned water containing active oxygen species and is capable of keeping the function of the active oxygen species for a long time contains first active oxygen species and second active oxygen species which are selected from a group consisting of superoxide anion radicals, hydroxy radicals, singlet oxygen and oxygen-containing organic radical species, one of the first active oxygen species and the second active oxygen species exhibits lower reactivity and is capable of keeping a function thereof for a longer time compared to the other active oxygen species, and in inducing the pathogen-resistance genes, the first active oxygen species is made fast-acting and, subsequently, the second active oxygen species is made slow-acting thus preventing diseases in plants. Accordingly, in inducing the pathogen-resistance genes, the fast-acting first active oxygen species is applied to plants which exhibit high sensibility to the active oxygen species thus preventing a disorder which may be caused by the second active oxygen species, and the second active oxygen species is applied to plants which exhibit low sensibility to the active oxygen species so that the pathogen-resistance genes can be introduced in a sustainable manner by the second active oxygen species whereby the water can surely express the pathogen-resistance genes to various plants. Further, in a plant pathogen-resistance inducing step (a prevention step) where it is unnecessary to directly apply the active oxygen species to pathogenic bacteria, it is possible to expect effects of the second slow-acting or gentle-acting active oxygen species. On the other hand, with respect to plant body in which the prevention stage has already passed and the infection of pathogenic bacteria advances, it is possible to selectively make use of the first active oxygen species for the purpose of directly sterilizing the pathogenic bacteria.

Further, according to the method of preventing diseases in plants called for in claim 5, the active oxygen species is produced by applying at least one of ultraviolet rays, ultrasonic oscillations, a visible light, microwaves to a catalytic body immersed into water. Accordingly, it is possible to produce the active oxygen species from the catalytic body with high efficiency.

Further, according to the method of preventing diseases in plants called for in claim 6, the water is water in which at least one of an oxygen gas, an ozone gas, a chloride gas, a nitrogen monoxide gas and an ammonium gas which constitutes the precursor of the active oxygen species is dissolved. Accordingly, the production efficiency of active oxygen species can be further enhanced.

Further, according to the method of preventing diseases in plants called for in claim 7, the catalytic body contains at least one selected from a group of metals constituted of metal oxide ions or metal hydroxide ions of titania (TiO₂), alumina(Al₂O₃), anodized aluminum, magnesium oxide, magnesium hydroxide, magnetite (Fe₃O₄), zinc oxide, tungsten oxide, barium titanate, strontium titanate, sodium titanate, zirconium dioxide, tungsten oxide, a tungsten hydroxide compound, α-Fe₂O₃, cadmium sulfide, zinc sulfide, platinum, copper, palladium. Accordingly, it is possible to surely produce the active oxygen species in water.

Further, according to the method of preventing diseases in plants called for in claim 8, the catalytic body has a powdery form, a granular form or a fibrous form. Accordingly, a contact area between water and the catalytic body can be increased and hence, the reaction efficiency can be enhanced.

Further, according to the method of preventing diseases in plants called for in claim 9, the active oxygen containing water is produced by an active oxygen containing water producing apparatus in which the ultraviolet-ray light source is arranged in water and the periphery of the ultraviolet-ray light source is surrounded by the photocatalytic body formed of a fibrous body with the fixed gap held between the ultraviolet-ray light source and the photocatalytic body whereby the water flow is formed of a first water flow which ascends and descends by passing the gap defined between the ultraviolet-ray light source and the photocatalytic body, the second water flow which ascends and descends by spirally passing on the outer periphery of the photocatalytic body, and the third water flow which flows into or flows out from the texture of the photocatalytic body. Accordingly, the water can contain active oxygen species with high efficiency and the active oxygen containing water can be supplied to many kinds of plants.

Further, according to the invention called for in claim 10, the active oxygen containing water producing apparatus used in the method of preventing diseases in plants according to any one of claims 2 to 9 includes: the ultraviolet-ray light source which is arranged in the vessel provided with the water supply port and the active oxygen containing water takeout port; and the photocatalytic body which is formed of the fibrous body and surrounds the periphery of the ultraviolet-ray light source with a predetermined gap held between the photocatalytic body and the ultraviolet-ray light source, wherein the water flow supplied to the inside of the vessels is formed of the first water flow which ascends and descends by passing the gap defined between the ultraviolet-ray light source and the photocatalytic body, the second water flow which ascends and descends by spirally passing on the outer periphery of the photocatalytic body, and the third water flow which flows into or flows out from the texture of the photocatalytic body. Accordingly, it is possible to provide the apparatus which can efficiently manufacture the water containing active oxygen species which can induce the expression of pathogen-resistance genes by inducing a redox reaction in plant cells without using a chemical as an effective content.

Further, according to the invention called for in claim 11, the active oxygen containing water producing apparatus used in the method of preventing diseases in plants according to any one of claims 2 to 9 includes: the vessel which is made of a material which allows the transmission of ultraviolet rays therethrough, is provided with the water supply port and the water discharge port and houses a catalytic body in the inside thereof; the ultrasonic irradiation part which irradiates ultrasonic waves in the flow direction of water which flows into the inside of the vessel from the water supply port; and the ultraviolet-ray irradiation part which is arranged on the periphery of the vessel and irradiates ultraviolet rays to the catalytic body from the outside of the vessel, wherein at least the portion of the catalytic body is arranged in the area where the irradiation area to which ultrasonic waves are irradiated from the ultrasonic irradiation part and the irradiation area to which ultraviolet rays are irradiated from the ultraviolet-ray irradiation part overlap with each other. Accordingly, it is possible to provide the apparatus which can efficiently manufacture the water containing active oxygen species which can induce the expression of pathogen-resistance genes by inducing a redox reaction in plant cells without using a chemical as an effective content.

Further, according to the invention called for in claim 12, the active oxygen containing water producing apparatus further includes the microwave irradiation part which, when water which flows into the vessel from the water supply port flows toward the water discharge port, irradiates microwaves in the direction orthogonal to the direction along which the water flows in the inside of the vessel, wherein at least the portion of the catalytic body is arranged in the area where an irradiation area to which microwaves are irradiated from the microwave irradiation part, the irradiation area to which ultrasonic waves are irradiated from the ultrasonic irradiation part and the irradiation area to which ultraviolet rays are irradiated from the ultraviolet-ray irradiation part overlap with each other. Accordingly, it is possible to provide the apparatus which can more efficiently manufacture the water containing active oxygen species which can induce the expression of pathogen-resistance genes by inducing a redox reaction in plant cells without using a chemical as an effective content.

### Brief Description of the Drawings

[Fig. 1]
   A cross-sectional view of an apparatus for producing active oxygen water according to an embodiment as viewed in a side view.
[Fig. 2]
   A cross-sectional view of the apparatus for producing active oxygen water according to the embodiment as viewed in a plan view.
[Fig. 3]
   A schematic explanatory view of an apparatus for producing active oxygen water according to another embodiment.
[Fig. 4]
   A graph showing a change of spikes of KO₂ which constitutes makers of the superoxide anion radicals and a change of concentration of superoxide anion radicals produced in this embodiment.
[Fig. 5]
   A graph showing a change with time of singlet oxygen.
[Fig. 6]
   A graph showing a result of measurement of ozone concentration.
[Fig. 7]
   A graph showing a result of experiment using ozone water having initial concentration of 2.5ppm.
[Fig. 8]
   A graph showing a result of an inhibiting experiment by Tiron and DABCO.
[Fig. 9]
   An agarose electrophoresis photograph showing a result of expression of tobacco PR1a genes.
[Fig. 10]
   An agarose electrophoresis photograph showing a result of expression of tomato PR-1 genes.
[Fig.11]
   A schematic view of a state where a pulse-like stimulus of singlet oxygen and superoxide anion radicals are shown.

### Mode for carrying out the Invention

The present invention is an invention which is found in the course of verifying advantageous effects of water containing active oxygen species in producing such water containing active oxygen species, and is characterized in that active oxygen species is specifically produced and the produced active oxygen species is suitably supplied to a plant body so that the expression of pathogen-resistance genes is induced in the plant body thus allowing the plant body to obtain the systemic acquired resistance (hereinafter, referred to as "SAR"). The SAR is known as a defense reaction against various kinds of pathogenic microorganism such as viruses, bacteria and fungus, and the plant body can acquire the SAR by a systemically expressing a group of PR genes. Particularly, the active oxygen containing water according to this embodiment contains long-life super oxide anion radicals thus effectively inducing the expression of the pathogen-resistance genes in plants.

Originally, the plants potentially have the immune-defense mechanism which enhances the resistance against the intrusion of pathogens. For the purpose of suppressing the occurrence of diseases thus eventually restricting a use amount of agricultural chemical such as a sterilizer by making plants express immunity stimulating substances which can induce such potential resistance, attempts have been made to find the expression of genes using pathogen resistance inducing type chemicals which can enhance the resistance of target plants against pathogen microorganisms without directly affecting the pathogen microorganisms and, further, resistance imparting type breeding using molecular genetics such as the preparation of pathogen resistance genes excessive expression plant body based on genetic modification has been carried out.

In the course of such attempts, a path along which plants acquire an immune ability starting from a salicylic acid has been clarified (Salicylic Acid as a Defense-Related Plant Hormone, T. Kawano and T. Furuichi, SALICYLIC ACID A plant hormone S. Hayat and A. Ahmad 2007 Springer), and there has been known a path along which pathogen resistance genes are expressed as an initial reaction by way of superoxide anion radicals (O₂^{·-}), and the systematic acquired resistance (abbreviated as SAR in general) in which a post reaction occurs upon receiving the expression of pathogen resistance genes so that the whole plant body acquires the immune ability. As an index for the salicylic-acid-induced systematic acquired resistance, the expression of a group of PR genes represented by acid PR-1 genes (PR-1a) is used. By making use of the above-mentioned salicylic-acid-dependent resistance inducing path, it is possible to achieve not only the elimination of pathogenic microorganisms of plant and pathogenic fungus of plant but also the prevention of infection of pathogenic viruses of plant which cannot be eliminated with conventional agricultural chemicals such as a sterilizer.

Based on such understanding, there have been developed chemicals which induce a salicylic acid in a plant body and chemicals which imitate an action of a salicylic acid in a plant body. As chemicals which are currently mainly used, benzo(1,2,3)thiadiazole-7-carbothioic acid S-methyl ester (BTH) made by E.I. du Pont de Numours and Company (USA) and probenazole (product name: oryzemate) made by MEIJI SEIKA KAISHA, LTD. can be named. These chemicals have been popularly used because of being effective as a preventive agent for microbacterial diseases such as rice blast, bacterial leaf blight, glume disease, rice disease injuries, and as a preventive agent for bacterial diseases of cucumbers, lettuces, cabbages, broccolis, Chinese cabbages, leeks and the like.

However, due to the use of chemicals, there exists a possibility that, in a farming season where a large quantity of chemical is used, the chemical dissolves into a drainage canal and rivers and an amount of dissolved chemical exceeds a prescribed standard. Further, as an example where the increase of a load imposed on an environment is anticipated, on a data sheet of a commercially available probenazole (Hokho: lateral-row Oryzemate granular wettable powder), a caution on fish toxicity (LC50 value (48 hours) for carp: 8ppm) is made.

Active oxygen species is widely distributed in nature and in living bodies. It is known that strong acidic power of the active oxygen species possesses sterilization ability which causes cell membrane disorder. The utilization of active oxygen species in air purifiers and the like has been attracting attentions. Originally, active oxygen species include, in general, oxygen species such as superoxide anion radicals (O₂^{·-}), hydrogen peroxide water, hydroxy radicals (·OH) and singlet oxygen (¹O₂). Further, active oxygen species include, in broad meaning, lipid peroxide (LOOH, LOO·), oxygen halide (C1O⁻). Still further, active oxygen species include nitrogen monoxide radicals (NO.) or the like which is identified as an in-vivo vascular endothelial derived relaxation factor. The existence of active oxygen species is found as an attribute substance of a vascular endothelial cell disorder and active oxygen species is treated as an in-vivo disorder transmitting substance. The characteristics of active oxygen species have been clarified in the course of studies for finding out causes of this disorder, and it has been clarified that superoxide dismutase (SOD) and NO. play a role of erasing O₂^{·-} in a living body and performs the maintenance of homeostasis.

It has been reported recently that a minute amount of active oxygen species is expressed when a plant is germinated. The role of active oxygen species in the environment is still in the course of clarification, and an in-vitro measuring method and an in-vitro quantity determination technique of active oxygen species have been developed. Because of instability of active oxygen species, the lifetime or activity of active oxygen species can be held only for an extremely short time of approximately several milliseconds or less and hence, the constant and quantitative generation of active oxygen species in water has been considered impossible except for hydrogen peroxide.

Although hydrogen peroxide belongs to a category of active oxygen species in broad meaning, hydrogen peroxide is an extremely stable compound and can be preserved for a long period at room temperature, and can be generated with high concentration and hence, hydrogen peroxide is apparently different from other active oxygen species. However, as in the case of the measurement of the concentration of hydrogen peroxide which can be easily performed by a luminal reaction using an extremely minute quantity of hydrogen peroxide, hydrogen peroxide has been incorporated or used as a stimulus transmitting substance having general purpose availability in the clarification of an oxidizing/reducing reaction, that is, a redox reaction in the ecosystem or in the living body.

Further, the confirmation of the expression of genes has been experimentarily carried out in such a manner that a salicylic acid or hydrogen peroxide which is induced in plant cells is directly supplied to the plant body or plant cells experimentarily, and oxidation stress is applied to the plant body or the plant cells from the outside. However, to actually confirm the expression of the genes, it is necessary to supply a chemical of extremely high concentration such as a salicylic acid of 0.1 to 0.5mM or more or hydrogen peroxide of 2 to 10mM or more to the plant body or the plant cells. When the chemical is actually supplied to the plant with the concentration which largely exceeds the stimulus concentration which takes place in a living plant body originally, not only the plant body per se is injured thus locally leading to the death of cells but also some chemical is not taken into and remains in soils or water leading to problems on a load imposed on the environmental. Accordingly, it is impossible to use such a chemical at an actual agricultural site.

As another method, there is a method in which genes are transduced into the plants so that the plants can acquire pathogen resistance. However, with respect to plant breeding by the transduction of the genes into plants, it is necessary to verify stability and safety of genes in the long term in view of the influence which the transduction of the genes exerts on an ambient ecological system and a human body. It is mandatory for such breeding not to influence other plants due to horizontal diffusion of genes or the like. Further, it is also mandatory for such breeding to continuously verify that there is no such horizontal diffusion of genes. Accordingly, the plant breeding by the transduction of the genes has many tasks to be solved such as the limitation on the use of such plants, the restriction on import and export of such plants and the like before acquiring the approval of the cultivation of such plants in an agricultural field. In this manner, there are many obstacles and drawbacks to be solved before the plant is used as a food material.

Currently, in water treatment techniques, the sterilization of bacteria and the elimination of microorganisms in water are mainly performed by the irradiation of ultraviolet rays and the ozone aeration. Although ozone which is dissolved in water is vaporized and is diffused in the atmosphere as residual ozone, the residual ozone is unstable and hence, it is considered that the residual ozone does not impose a burden on the environment. However, the residual ozone continuously influences the environment in a stable state for several hours. Although the influence exerted by ozone is often confused with the acceleration of global warming caused by the destruction of the ozone layer in the stratosphere, ozone is heavier than carbon dioxide and ozone generated on the ground does not reach the stratosphere thus accelerating the global warming. There has also been a report that the troposphere ozone per se is a gas with a strong greenhouse effect and hence, 20% to 30% of the global warming brought about by the whole greenhouse effect gas is derived from a forced radiation force of the troposphere ozone (H. Akimoto and K. Sudo. Climate Sensitivity of Ozone. In : Air pollution and its relations to climate change and sustainable development, 2. Climate change and air pollution, 12-14 March, 2007, Gothenburg, Sweden; http://asta.iv1.se/Workshops/). Further, according to a recent report, there has been reported a simulation result that when the secondary influence exerted on the vegetation which is expected to absorb carbon dioxide is taken into consideration in addition to the direct greenhouse effect caused by ozone, the greenhouse effect derived from the troposphere ozone becomes twice as large as the conventionally expected greenhouse effect (Nature. 2007; Vol.448, No. 7155: pp.791-794). Accordingly, there has been a demand for the development of novel technology which can readily dissipate the residual ozone thus reducing a total ozone discharge quantity.

Under such circumstances, according to the present invention, it is also possible to eliminate pathogenic viruses of plant which cannot be eliminated in general with an agricultural chemical such as a sterilizer in the same manner as the elimination of bacteria or fungus and hence, the pathogen microorganism elimination effect can be realized in a wider range compared to the pathogen microorganism elimination effect obtained using the conventional agricultural chemical. Further, by selectively producing the active oxygen species, it is also possible to directly eliminate pathogen microorganisms which are already adhered to plants and cannot be eliminated with a resistance inducing type chemicals (BTH, probenazole or the like). Accordingly, it is possible to largely reduce the use of a chemical in an agricultural site where the use of a large number of chemicals is required.

As a means which brings such active oxygen containing water into contact with plants (plant contacting means), for example, a method in which hydroponic solution into which the plants are immersed is formed using active oxygen containing water, a method in which active oxygen containing water is impregnated into soils where the plants grow, or a method in which active oxygen containing water is directly applied to the plants can be named. Due to the water absorbed by the plants in this manner, a redox reaction is induced in the plant thus expressing pathogen-resistance genes (PR gene group) to encode plant stimulating immunoproteins (pathogenesis-related protein). Here, the active oxygen containing water is desirably brought into contact with the plant body directly without forming the active oxygen containing water into a gaseous form or in a mist form. When the active oxygen containing water is formed into a gaseous form or in a mist form, the active oxygen species acts on organic substances or floating bacteria in the atmosphere and is consumed and hence, a detection half life of the active oxygen species is shortened (although the active oxygen species per se exists for an extremely short time, in the produced active oxygen containing water, the reaction of the active oxygen species is continuously performed and hence, the active oxygen species is detected as if the active oxygen species has the half life). Accordingly, the concentration of active oxygen species which reaches and acts on the plant body is low and hence, the efficient expression of pathogen-resistance genes becomes difficult.

Originally, when ultrasonic oscillations are applied to the catalytic body, energy generated due to the ultrasonic oscillations on a gas-liquid boundary surface (water surface) is consumed for atomizing water or is consumed by impinging on internal substances of the catalytic body or by peeling off organic substances or the like adhered to the catalytic body.

However, according to one technical feature of the present invention, in a closed reaction vessel (in a closed space), energy generated by ultrasonic oscillations is not dissipated and is consumed in water and hence, the energy directly acts on oxygen atoms dissolved in water thus generating active oxygen species such as superoxide anion radicals or singlet oxygen.

Further, according to another technical feature of the present invention, by arranging the catalytic body in the inside of the closed reaction vessel, a catalytic reaction which is caused by bringing water or running water into contact with the catalytic body is brought about by applying ultrasonic oscillations to the catalytic body and by irradiating electromagnetic waves to the catalytic body thus generating active oxygen species. A series of these reactions increases the generation of active oxygen species in water caused by the above-mentioned ultrasonic oscillations and brings about a synergistic effect.

Here, active oxygen species is generally called as superoxide, and includes superoxide anion radicals (O₂^{·-}), hydroxy radicals (·OH) , singlet oxygen (¹O₂), halogenation oxygen (XO⁻, hypochlorite ions (C1O⁻)as an example), nitrogen monoxide radicals (NO·), peroxy nitrate (ONOO^{-·}), organic radicals containing oxygen (phenoxy radicals as an example), excluding hydrogen peroxide water(H₂O₂) due to properties thereof.

To allow water to contain active oxygen species as much as possible therein, conventionally, there has been known a method where ultraviolet rays are irradiated to a photocatalyst immersed in water thus diffusing the active oxygen species generated on a surface of the photocatalyst in water. According to this method, however, when the ultraviolet-ray permeability of water is low (for example, when suspensions are present in water or when water contains ultraviolet-ray absorbing substances), ultraviolet rays which are radiated from an ultraviolet rays source are attenuated before ultraviolet rays radiated from the ultraviolet rays source reach the photocatalyst by permeating water and hence, there may be a case where a generation quantity of active oxygen species becomes extremely small.

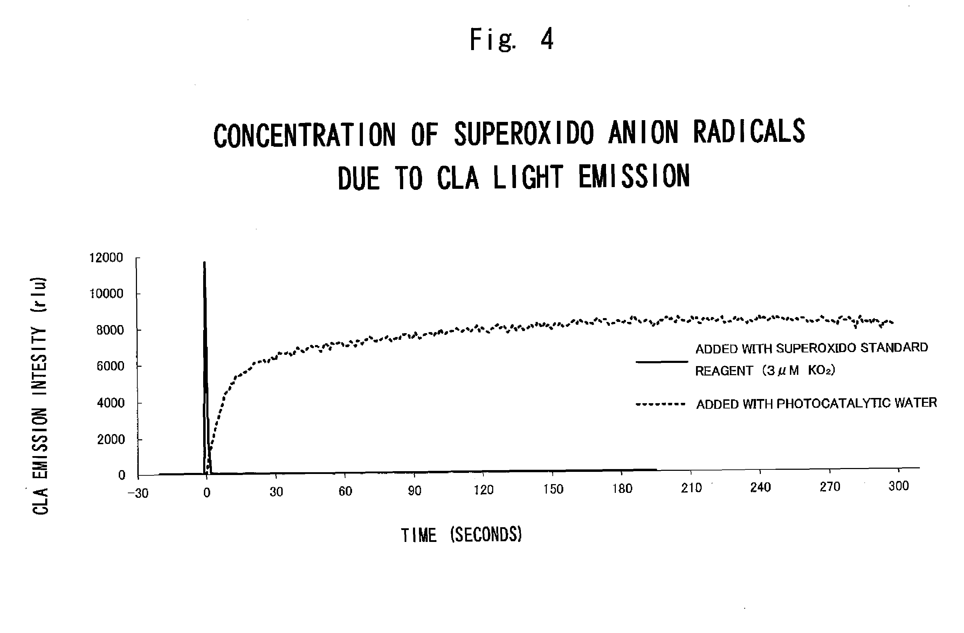

Further, it is considered in general that active oxygen species which is present in water has a short lifetime. Potassium superoxide KO₂ which is a standard reagent of superoxide is used as a quantitative standard reagent, and KO₂ potassium superoxide is provided as a marker, for example. However, KO₂ is a chemical which has extremely unstable physical properties so that KO₂ is dissolved in DMPO in transporting KO₂ and handling of KO₂ requires careful attention as an explosive substance. Further, the lifetime of KO₂ is extremely short, that is, KO₂ is extinguished within several milliseconds after being dropped into water so that the emission of light is not observed for 2 seconds or more (shown in Fig. 4 as a marker).

The extinction of KO₂ within an extremely short several milliseconds in water is the main reason that, in causing a redox reaction in the plant body which induces the expression of pathogen-resistance genes, a probability that active oxygen species and redox response molecules on surfaces of cells are brought into contact with each other and generate the redox reaction is extremely lowered.

Accordingly, there has been a demand for producing water containing active oxygen species which can contain a sufficient amount of active oxygen species and allows the active oxygen species to keep its function for a longer time.

To satisfy such a demand, the inventors of the present invention have established a technique in which a catalytic body is arranged in water and, as a means for generating active oxygen species from the catalytic body in water, ultrasonic oscillations, ultraviolet rays, a visible light and microwaves are applied to a metal oxide catalytic body so that water containing a large quantity of active oxygen species is produced by a single effect or a combined or synergistic effect and, further, super oxide anion radicals, singlet oxygen or the like which are active oxygen species is selectively produced.

Water which is brought into contact with the catalytic body may be running water. By bringing running water into contact with the catalytic body, running water downstream of the catalytic body becomes water containing a large quantity of active oxygen species so that it is possible to continuously produce water containing active oxygen species.

Here, as a typical example which generates such reactions, a process where active oxygen species is generated in water is explained by taking a photocatalytic reaction which is caused by an ultraviolet-ray light source and titanium oxide as examples.

Usually, in a case of ionized water H₂O, oxygen is always dissolved in water except for a peculiar state, and the following reaction is accelerated. However, energy (hv) derived from ultraviolet rays excites metal oxide atoms (XₙOₘ, for example, such as TiO₂, Al₂O₃ or the like) on a surface of the metal oxide film so that free electrons and holes (photon) are generated, and it is thought that the following reaction takes place.

XₙOₘ (for example, TiO₂) + hv → e⁻ + h+VB

h+VB → h+tr

O₂ + e⁻ → O₂·⁻

O₂·⁻ + h+VB (h+tr) → O₂

OH⁻ + h+VB → OH·

When water contains a large quantity of oxygen, these reactions are accelerated so that a large quantity of O₂·⁻(superoxide anion radicals) and OH-(hydroxy radicals) are generated.

Here, when water contains chlorine ions, it is thought that the following reaction is activated.

2Cl⁻ + O₂+ e⁻ → 2ClO⁻

Further, when water contains a large quantity of ozone, it is thought that the following reactions are activated.

O₃ + hv → O(1D) + O₂ (a¹Δ_{g})

O₃ + h+VB (h+tr) → O₂·-

The above-mentioned reactions usually do not efficiently progress with the direct excitation of ozone with the radiation of ultraviolet rays in a UV-A region. However, it is thought that under the presence of the photocatalytic body which is in a light-dependent excited state, the above-mentioned reactions which also bring about the generation of singlet oxygen progress.

The above-mentioned formulae are also expressed by the following three formulae.

OH⁻ + h+VB → OH·

OH· + O₃ → O₂+ HO₂

HO₂ ⇔ H⁺ + O₂^{·-}

The increase of the generation of super oxide anion radicals and the increase of the generation of singlet oxygen using ozone water as a precursor substance are, as described later, verified by the inventors of the present invention using chemical luminescence of Cypridina luciferin analog derived from sea fireflies. The generation of hydroxy radicals which appears in these reactions is also verified by the inventors of the present invention using an electron spin resonance method which makes use of 5.5-Dimethyl-1-pyrroline N-oxide (DMPO) as a spin trapping agent.

Further, when water contains a large quantity of nitrogen monoxide gas, ions having strong cellular cytotoxicity are generated due to following reactions.

NO + e⁻→ ·NO

NO - e⁻ → NO₂⁻

2NO₂⁻ - 2e⁻ → 2·NO₃⁻

·NO + NO₂⁻ → N₂O₃

·NO + O₂ → ·ONOO⁻

·ONOO⁻ + H⁺ → HOONO → OH·

The generation of O₂·⁻ , the generation of ·NO, the generation of ·ONOO⁻ and the generation of OH· which take place here are already verified by experiments carried out by the inventors of the present invention using a reaction with fluorescence probes, oxidation assay of folic acid (fluorescence method) and an electron spin resonance method.

Further, these reactions are a group of reactions which are caused only due to the movement of electrons and hence, these reactions are easily reversible whereby the generated secondary reaction ions return to a state before the secondary reaction. Accordingly, active oxygen species which originally has an extremely short lifetime is continuously generated so that it is possible to make active oxygen species act as if the active oxygen species which have a half life of activity is generated is generated.

Further, in this specification, the above-mentioned water containing active oxygen species which allows the plant body to acquire pathogen-resistance genes implies water containing active oxygen species such as super oxide anion radicals or singlet oxygen excluding hydrogen peroxide at the initial concentration of 1µmol per 1L or more. However, at this point of time, there is no marker which simply prescribes the concentration of singlet oxygen and hence, it is assumed that the concentration of singlet oxygen implies titer corresponding to the concentration in terms of the concentration of super oxide anion radicals or the concentration of ozone water.

Assume that the initial concentration of active oxygen species is less than 1µmol/L, a redox reaction sufficient for allowing the plant body to acquire pathogen-resistance genes is not induced so that such water containing active oxygen species is not practically used.

Further, in the above-mentioned water containing active oxygen species, by making use of a phenomenon that Cypridina luciferin analog derived from sea fireflies selectively reacts with super oxide anion radicals and singlet oxygen and exhibits quantitative blue chemical luminescence, it is possible to detect super oxide anion radicals (a technique publicly proposed by the inventors of the present invention in Bioluminescence & Chemiluminescence, 2008). Further, the activity of super oxide anion radicals is suppressed with the addition of Tiron which is superoxide removing agent, and super oxide anion radicals are differentiated from singlet oxygen due to an effect of DABCO which is a reagent for selective removing singlet oxygen. Using the method according to the present invention, it is possible to verify that an effect of water containing active oxygen species can be surely obtained outside the apparatus for producing water, and it is also possible to verify that active oxygen species is diffused in water and hold activity thereof so that the reaction continues.

The catalytic body used in the apparatus for producing water containing active oxygen species is not particularly limited in shape, and may be formed into any one of particles, grains, beads and fibers. A surface of each catalytic body is covered with a metal oxide film having a catalytic function.

Here, a material of fibers is not particularly limited, and may be glass, ceramic or non-woven fabric, for example.

A suitable known method may be used to form a metal oxide film having a catalytic function on a surface of the fibers which is made of such a material. For example, a dip coating method may be used.

The metal oxide film formed in this manner may be mainly made of metal oxides such as alumina, aluminum hydroxide, titanium oxide, magnesium oxide, magnesium hydroxide, zinc oxide, tungsten oxide, barium titanate, strontium titanate, sodium titanate, zirconium dioxide, tungsten oxide, hydroxide tungsten compound, α-Fe₂O₃, cadmium sulfide, zinc sulfide, platinum, copper and palladium.

By forming the metal oxide film made of these materials, it is possible to efficiently generate active oxygen species in water or running water.

Further, the fibers may be formed of aluminum fibers, and an alumina coating film having a thickness of 30nm or more may be formed on a surface of the aluminum fibers by sintering.

Not to mention that the catalytic body which is formed by collecting the aluminum fibers having such an alumina coating film can efficiently produce active oxygen species, the catalytic body may be formed of a photocatalytic body which is formed by applying a titanium oxide film to the aluminum fibers by coating using a dip coating method.

Further, the apparatus for producing water containing active oxygen species may be configured such that a light source of ultraviolet rays which are a kind of microwaves is arranged in water, a catalytic body formed of fibers surrounds the periphery of the ultraviolet ray light source with a fixed gap therebetween whereby a water flow is formed of a first water flow which ascends or descends by passing through a gap defined between the ultraviolet ray light source and the catalytic body, a second water flow which ascends or descends by spirally passing over the outer periphery of the catalytic body, and a third water flow which flows into or flows out from the tissues of the catalytic body.

Although explained specifically in detail later in conjunction with the drawings, by forming the first water flow which ascends or descends by passing through the gap defined between an inner peripheral surface of the cylindrical catalytic body and the ultraviolet ray light source and the second water flow which circulates spirally on the outer surface of the catalytic body, it is possible to uniformly bring the water flow into contact with the catalytic body. Further, the catalytic body is formed as a fiber collective body and hence, it is possible to form the third water flow which passes through the inside of the catalytic body from the first water flow and is merged to the second water flow whereby water in the inside of the catalytic body is allowed to flow effectively thus realizing the more effective generation of active oxygen species.

To explain advantageous effects obtained by the ultrasonic oscillator used here, the ultrasonic oscillator performs not only the generation of electrons and holes by a catalytic reaction excited on the catalytic body but also minute oscillations of the catalytic body. Due to such minute oscillations of the catalytic body, the generation of active oxygen species generated by a photocatalytic reaction can be smoothly conducted thus allowing the active oxygen species to float in water freely. When the catalytic body is formed of a fibrous body, although a relative position of the fibrous catalytic body is fixed in the inside of the apparatus, respective fibers have free ends and are brought into contact with each other in an entangled manner thus maintaining a shape of the catalytic body.

That is, with respect to a flow speed of water which flows on a surface of the fibers, the fibers move at an ultra high speed due to ultrasonic oscillations and hence, an interface boundary flow speed is remarkably enhanced thus realizing the discharge of active oxygen species into water.

That is, these ultrasonic waves accelerate the separation of active oxygen species from the catalytic body and, at the same time, the reaction is amplified due to a mutual interference action in wavelength between ultrasonic waves and ultraviolet rays.

With respect to the ultrasonic oscillator, for example, the use of atomization ultrasonic oscillator (high-frequency ultrasonic oscillator) which generates high-frequency ultrasonic waves (in general, 500 kHz or more) is recommended.

The high-frequency ultrasonic oscillations generated by this atomization ultrasonic oscillator exhibits low catalytic body cleaning ability. However, the strong cavitation energy of the ultrasonic oscillations directly acts on oxygen atoms dissolved in water thus bringing about not only the generation of the active oxygen species but also the generation of a catalytic reaction on a metal oxide film, and also has sufficient power of a level which scatters the electrons, active oxygen species and the like generated due to the catalytic reaction into water.

Further, the middle-frequency ultrasonic waves (101 to 500kHz) may be used as the ultrasonic waves. In using the middle-frequency ultrasonic waves generated by the middle-frequency ultrasonic oscillator, when the ultrasonic waves impinge on the catalytic body, the diffraction property of sound waves is increased so that the agitation of water in a closed vessel is further enhanced whereby active oxygen species is efficiently separated from the catalytic body. In this case, it is deniable that the deterioration of the catalytic body is increased compared to the high frequency oscillator. However, an action of the middle-frequency ultrasonic waves can bring about an effect of cleaning substances having a relatively large molecular weight such as turbid components adhered to the catalytic body.

However, the low-frequency ultrasonic waves of 100kHz or less may cause the deformation of the catalytic body or peeling or damage on a catalyst reaction surface formed on the catalytic body, and the active oxygen species generation efficiency is also poor and hence, the use of the low-frequency ultrasonic waves is not so desirable.

Further, it may be also possible to produce a reactant which reacts with the desired active oxygen species by preliminarily mixing a predetermined gas into water or running water which is brought into contact with the catalytic body as described previously. With respect to the adjustment of the gas concentration, it may be possible to adjust the concentrations of dissolved oxygen and hydrogen by decomposing water.

That is, in a state where a catalytic function of the catalytic body is not still imparted to water or running water, an oxygen gas, an ozone gas, a chlorine gas, a nitrogen monoxide gas or an ammonia gas is mixed into water or running water so as to generate various active oxygen species such as super oxide anion radicals, hydroxy radicals, singlet oxygen, hydrogen peroxide water (H₂O₂), hypochlorous acid ions (ClO⁻), nitrogen monoxide radicals (NO·) or peroxy nitrate (ONOO^{-·}) in water or running water.

Further, it may be also possible to control activity of active oxygen species by applying chemical treatment and/or physical treatment to water containing active oxygen species.

To be more specific, it is possible to control the activity of active oxygen species by adjusting additives, dissolved oxygen concentration, dissolved ozone concentration, temperature, pH, viscosity or the like in water or running water which is brought into contact with the catalytic body.

A quantity of active oxygen in active oxygen containing water is reduced with a fixed half-life as described previously. According to the present invention, by providing an active oxygen concentration control part to the apparatus for producing water containing active oxygen species, it is possible to change a decreasing speed of active oxygen species concentration.

Further, the inventors of the present invention have found that the concentration of dissolved oxygen in water is lowered along with the elevation of water temperature in water, and when water with increased dissolved oxygen concentration is left, the oxygen concentration is reduced with time but, at the same time, activity (concentration) and lifetime (activity expression holding time) of the active oxygen species is changed corresponding to a change in the dissolved oxygen concentration, temperature and pH in water. Further, the inventors of the present invention have clarified a phenomenon where 10⁻⁷ to 10⁻⁸% of oxygen dissolved in water usually always contains superoxide anion radicals, and also have found that the apparatus for producing water containing active oxygen species allows the active oxygen species to exhibit strong oxidation power by changing a content ratio of dissolved oxygen to 10⁻⁴ to 10⁻⁶%.

That is, the dissolved oxygen concentration, water temperature and pH are deeply relevant to the active oxygen species generating ability. For example, the following active oxygen concentration control method has been established. In this method, for example, water which is introduced into the apparatus for producing water containing active oxygen species is separated into acidic water and alkaline water in advance by an ionization decomposition method. Then, only alkaline water is cooled and oxygen is given to alkaline water and, thereafter, alkaline water is introduced into the apparatus for producing water containing active oxygen species. Then, alkaline water is introduced into a treatment vessel which is provided for the sterilization of bacteria, the elimination of microorganisms and the decomposition of organic substances, and treatment is applied to alkaline water for a fixed time. Thereafter, stored acidic water is added to alkaline water thus remarkably shortening lifetime of active oxygen species.

The above-mentioned control method and change of concentration are characteristic features when an oxygen gas is supplied to a gas adding device. The same phenomenon occurs when an ozone gas, a chloride gas, a nitrogen monoxide gas or an ammonia gas is used as a kind of addition gas.

Further, the active oxygen concentration control part may include, as a means which adjusts a decreasing speed of concentration of active oxygen species, a chemical means which performs treatment such as the adjustment of the dissolved ion concentration (for example, copper ions, aluminum ions, iron ions or the like), the adjustment of pH of an aqueous solution or addition of chemicals such as a superoxide remover (Tiron or the like) or a physical means such as a pressure reduction device in a gas phase or a water reservoir temperature control device.

Since the apparatus for producing water containing active oxygen species includes the active oxygen concentration control part, with respect to active oxygen containing water produced by the apparatus for producing water containing active oxygen species according to the present invention, during a period where the activity of active oxygen is requested, it is possible to slow down a decreasing speed of concentration of active oxygen species so as to maintain active oxygen at high concentration by holding a pressure in a gas phase at a high level or by holding a temperature of the water reservoir at a low level. Thereafter, it is possible to increase the decreasing speed of concentration of active oxygen species by reducing a pressure in gas phase using a vacuum pump or by elevating the temperature of the water reservoir to a high temperature.

In increasing the decreasing speed of concentration of active oxygen species, a active oxygen species removing agent such as Tiron or DABCO or a reducing agent such as a thiol group may be added to water containing active oxygen species so as to realize the rapid removal of active oxygen species.

Such an active oxygen concentration control brings about an advantageous effect that the activity of active oxygen containing water is increased only during a predetermined period and, thereafter, active oxygen species containing water is brought back to an inactive state to become ordinary water. With the use of the physical means, it is possible to minimize the influence of residual substances on an environment compared to a case where the chemical means is used. As the physical means, besides an atmospheric pressure operating means such as the vacuum pump or the water reservoir temperature control device described above, a water pump, a device which performs agitation by aeration, an electrically neutralizing device or the like is available.

By mounting the active oxygen concentration control part on the apparatus for producing water containing active oxygen species described in detail later as an attachment, the apparatus for producing water containing active oxygen species can acquire the compact constitution. However, the arrangement of the active oxygen concentration control part is not limited to the above arrangement and, for example, the apparatus for producing water containing active oxygen species and the active oxygen concentration control part may be provided separately from each other such that the active oxygen concentration control part is arranged in a water flow passage of active oxygen containing water.

Further, in addition to the previously-mentioned ultrasonic oscillator which is arranged on the apparatus for producing water containing active oxygen species and promotes the generation of active oxygen, an ultrasonic oscillator which generates ultrasonic waves for attenuating ultrasonic waves generated from the above-mentioned ultrasonic oscillator (hereinafter referred to as attenuation ultrasonic oscillator) may be arranged so as to adjust a generation quantity of active oxygen.

That is, the frequency of ultrasonic waves generated from the attenuation ultrasonic oscillator is set to a frequency which can attenuate energy generated from the ultrasonic oscillator by interference with the ultrasonic waves generated from the ultrasonic oscillator.

Due to such a constitution, it is possible to adjust a generation quantity of active oxygen by suitably adjusting an electric current which flows in the ultrasonic oscillator and an electric current which flows in the attenuation ultrasonic oscillator.

Further, since the generation quantity of active oxygen can be electrically adjusted, it is possible to adjust the generation quantity of active oxygen more finely compared to the adjustment using chemicals.

Hereinafter, embodiments of the present invention are explained in more detail in conjunction with examples.

### [Specific constitution of active oxygen containing water producing apparatus]

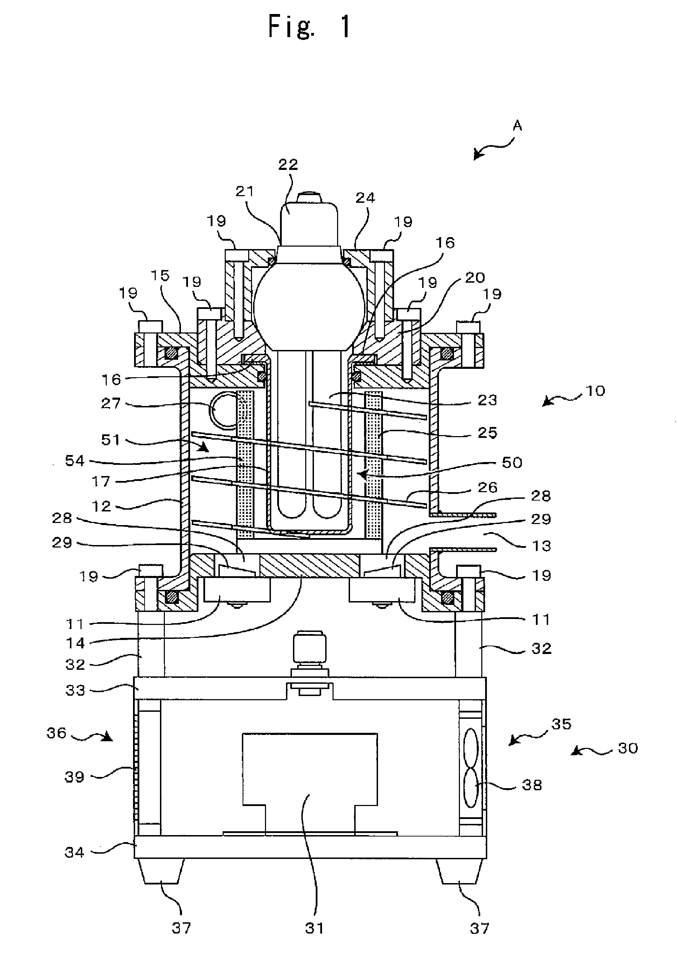

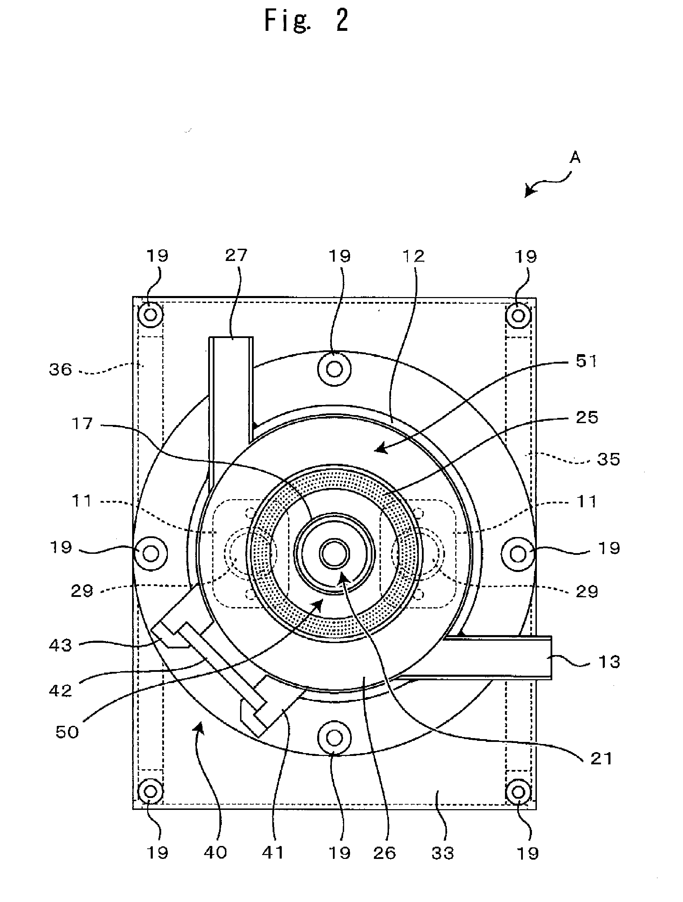

Firstly, the active oxygen containing water producing apparatus A according to this embodiment is explained in conjunction with Fig. 1 and Fig. 2. Fig. 1 is a cross-sectional side view of the active oxygen containing water producing apparatus A according to this embodiment, and Fig. 2 is a cross-sectional plan view of a reaction part 10 and the surrounding of the reaction part 10.

The active oxygen containing water producing apparatus A is constituted of a reaction part 10 which is a reaction vessel for producing water containing active oxygen species with the supply of water (running water) and is provided with water flow passages, and a control part 30 which controls driving of ultrasonic oscillators 11 mounted on the reaction part 10.

The reaction part 10 includes an approximately-cylindrical reaction-part outer sleeve 12. A water inlet 13 which constitutes a port for receiving water (running water) containing active oxygen species and a water outlet 27 which constitutes a port for discharging water containing active oxygen species is formed in the reaction-part outer sleeve 12. A bottom opening formed in the reaction-part outer sleeve 12 is closed by a reaction-part bottom plate 14, and an upper opening of the reaction-part outer sleeve 12 is covered with a cell-body engaging member 15.

Further, also as shown in Fig. 2, a naked-eye viewing window 40 is formed on an outer peripheral surface of the reaction-part outer sleeve 12 such that an operator can observe a state in the reaction-part outer sleeve 12.

It is needless to say that the formation of the naked-eye viewing window 40 is unnecessary depending on a usage of the water producing apparatus A.

The naked-eye viewing window 40 is formed as follows. A window frame body 41 in which a hole having a predetermined shape is formed is arranged in a partially cutaway portion of an outer peripheral surface of the reaction-part outer sleeve 12, a transparent plate 42 made of a transparent material such as glass or an acrylic resin is fitted into the window frame body 41 so as to close the hole, and the transparent plate 42 is fixed by a transparent-plate-pushing body 43 from the outside.

Firstly, an area in the vicinity of an upper portion of the reaction-part outer sleeve 12 is explained. The cell-body engaging member 15 has an approximately doughnut shape by forming a hole in a center portion thereof as viewed in a plan view, and the cell-body engaging member 15 is fixed to the reaction-part outer sleeve 12 using bolts 19.

Into the hole formed in the cell-body engaging member 15, a cup-shaped cell body 17 which is formed using a transparent material and has a flange portion 16 is fitted. To be more specific, the cell body 17 is fitted into the hole such that the cell body 17 is inserted into the reaction-part outer sleeve 12 through the hole formed in the center of the cell-body engaging member 15 and the flange portion 16 is engaged with a peripheral end portion of the hole of the cell-body engaging member 15.

The cell body 17 is made of a material having high transmission efficiency of the electromagnetic wave, and is made of silica glass, for example.

Further, a packing 18 formed of an elastic body is arranged on an inner wall surface of a hole formed in the cell-body engaging member 15. The packing 18 closes a gap defined between the inner wall surface of the hole formed in the cell-body engaging member 15 and the cell body 17 thus preventing water (running water) from leaking to an upper portion of the cell body 17.

Further, the flange portion 16 of the cell body 17 is sandwiched between a cell body pushing member 20 and the cell-body engaging member 15 thus restricting the upward movement of the cell body 17. Here, the cell body pushing member 20 is fixed to the cell-body engaging member 15 using the bolts 19.

Further, an electromagnetic-wave-generation-device insertion hole is formed in a center portion of the cell body holding member 20 so that the cell body holding member 20 has an approximately doughnut shape as viewed in a plan view. Here, an electromagnetic wave generation device 21 can be inserted into the electromagnetic-wave-generation-device insertion hole.

In the apparatus for producing water containing active oxygen species according to this embodiment, an ultraviolet ray tube (blacklight, effective wavelength of ultraviolet rays: 356nm) having a shape which is generally called as a bulb-shaped fluorescent lamp is shown as one example of an electromagnetic wave generation device 21. The electromagnetic wave generation device 21 may be a bactericidal lamp (effective wavelength of ultraviolet rays: 256nm) or a high luminance LED, and may be an electromagnetic wave generation device used in a microwave oven or the like.

By connecting a bulb socket or the like to an electricity supply part 22 formed on an upper portion of the electromagnetic wave generation device 21 and by supplying electricity to the electromagnetic wave generation device 21, electromagnetic waves are radiated from the electromagnetic wave generation device 21.

Further, the electromagnetic wave generation device 21 is inserted into the electromagnetic-wave-generation-device insertion hole thus allowing a tube part 23 of the electromagnetic wave generation device 21 to face the inside of the cell body 17 whereby the electromagnetic waves can be radiated to the inside of the reaction-part outer sleeve 12 through the cell body 17.

Further, the electromagnetic wave generation device 21 is fixed to the cell body holding member 20 using an electromagnetic-wave-generation-device fixing member 24, and the cell body holding member 20 and the electromagnetic-wave-generation-device fixing member 24 are fixedly connected to each other using bolts 19.

Due to such a constitution, for example, even in a state where water is supplied to the active oxygen containing water producing apparatus A, it is possible to exchange the electromagnetic wave generation device 21 without stopping the supply of water.

Next, the inner structure of the reaction-part outer sleeve 12 is explained. A catalytic body 25 which is formed in a cylindrical shape and surrounds an outer peripheral surface of the cell body 17 is arranged in the inside of the reaction-part outer sleeve 12, and a helical plate 26 which is formed into a helical shape along an inner wall surface of the reaction-part outer sleeve 12 is formed around an outer periphery of the catalytic body 25.

A first water flow passage 50 which allows water to flow in the vertical direction is formed between the outer peripheral surface of the cell body 17 and the inner peripheral surface of the catalytic body 25.

Further, in a space defined between the outer peripheral surface of the catalytic body 25 and the inner peripheral surface of the reaction-part outer sleeve 12, the helical plate 26 is formed such that the helical plate 26 turns around the outer peripheral surface of the catalytic body 25 in plural turns thus forming a second water flow passage 51 which constitutes a flow passage for a second water flow in which water flows in a spirally elevated manner along the helical plate 26 between plates which overlap each other in the vertical direction.

The catalytic body 25 is formed of a mass of fibers each of which has a diameter of approximately 50 to 200µm (100µm in this embodiment) and is formed of a collective body formed of metal fibers. Kinds or quantities of fibers can be changed corresponding to the usages. For example, the fibers may be formed such that aluminum fibers are formed by sintering an alumina film on a surface of the aluminum fibers, and by covering surfaces of the formed alumina film with a titanium oxide.

Particularly, the catalytic body 25 used in this embodiment is formed of fibers having free ends thereof vibrated by ultrasonic oscillations, and by applying ultrasonic oscillations to the catalytic body 25, the free ends of the fibers of the catalytic body 25 are vibrated so that it is possible to increase a flow speed of water which flows while being in contact with a surface of the catalytic body 25 whereby it is possible to disperse a large quantity of active oxygen species in water which flows on the surface of the catalytic body 25. Here, "free ends" is a term which implies respective end portions of fibers of a collective body formed of fibrous substances.

To be more specific, active oxygen species is generated from of a surface of the catalytic body 25 having a vast surface area which is a mass of front surfaces of respective fibers and, at the same time, the generated active oxygen species is readily separated by shaking from the surface of the catalytic body 25 due to ultrasonic oscillations, and a large quantity of active oxygen species freely float in water.

Then, new active oxygen species is readily generated on the surface of the catalytic body 25 and, again, the active oxygen species is separated by shaking from the surface of the catalytic body 25 due to ultrasonic oscillations and freely float in water.

This action is repeated instantaneously and frequently and hence, it is possible to allow water to contain active oxygen species extremely efficiently.

Further, the catalytic body 25 formed of fibers having a diameter of 50 to 200µm can generate oscillations more easily in conformity with minute ultrasonic oscillations compared to a plate-shaped catalytic body thus enabling the easier separation of the active oxygen species from the surface of the catalytic body.

Further, distal end portions of a large quantity of metal fibers constituting the catalytic body 25 behave as free ends under ultrasonic oscillations and hence, it is possible to efficiently separate active oxygen species into water from the catalytic body.

That is, to efficiently disperse active oxygen species generated by the catalytic body 25 into water, the catalytic body 25 is formed of fibers having free ends, and a relative speed between the free ends of the catalytic body 25 and the water flow is remarkably increased by giving ultrasonic oscillations and hence, it is possible to produce active oxygen containing water.

Further, a function of active oxygen species can be imparted to water, and this water can be discharged or taken out from the active oxygen containing water producing apparatus A as active oxygen containing water.

In this manner, corresponding to a precursor substance of active oxygen species dissolved in water, it is possible to separate a large quantity of superoxide anion radicals or singlet oxygen. Accordingly, even after being taken out from the active oxygen containing water producing apparatus A, active oxygen containing water can continuously maintain active oxygen species for a long time so that active oxygen containing water can induce pathogen-resistance genes which the plant body possesses.

To further explain the present invention by returning to Fig. 1 and Fig. 2, the catalytic body 25 allows water to pass through between the first flow passage 50 and the second flow passage 51, and a flow passage for a third water flow which passes through the catalytic body 25 is referred to as a third flow passage 52.

Here, a starting end portion of the second flow passage 51, that is, a lower end portion of the helical plate 26 is arranged to face a water inlet 13 formed in a lower portion of an outer peripheral side surface of the reaction part outer sleeve 12 as shown in Fig. 2 which is a cross-sectional view.

Due to such a shape, water supplied to the reaction generation vessel 73 through the water inlet 13 can more easily flow into the second flow passage 51 than the first flow passage 50 so that the pressure difference attributed to the difference in flow speed is generated between the first flow passage 50 and the second flow passage 51.

Accordingly, it is possible to efficiently generate the third water flow in the third flow passage 52 formed in the catalytic body 25 and hence, it is possible to allow water (running water) to effectively contain active oxygen species generated by the catalytic body 25 therein.

Then, water containing active oxygen species (running water) can be discharged and taken out through a water outlet 27 formed on an upper portion of an outer peripheral surface of the reaction part outer sleeve 12.

Next, the structure of the reaction generation vessel 73 in the vicinity of the lower portion of the reaction part outer sleeve 12 is explained. A bottom portion opening formed in the reaction part outer sleeve 12 is closed by a reaction part bottom plate 14. Exposure holes 28 which expose a part of the ultrasonic oscillators 11 in the inside of the reaction part outer sleeve 12 are formed in the reaction part bottom plate 14.

A plurality of (two in this embodiment) ultrasonic oscillators 11 are arranged on a lower portion of the reaction part bottom plate 14, and expose oscillation plates 29 of the ultrasonic oscillators 11 toward the inside of the reaction part outer sleeve 12 from the above-mentioned exposure holes 28. Due to such a constitution, ultrasonic oscillations are imparted to water (running water) filled in the reaction part outer sleeve 12 or to the catalytic body 25.

Further, the oscillation plates 29 arranged on the ultrasonic oscillators 11 are arranged obliquely at a predetermined angle with respect to the horizontal direction so that ultrasonic oscillations are efficiently imparted to the catalytic body 25.

Next, the control part 30 which is arranged below the reaction part 10 is explained.

The control part 30 and the reaction part 10 are joined to each other by way of supports 32.

The control part 30 has a box shape and incorporates an ultrasonic oscillation generating device 31 therein. To be more specific, the control part 30 includes a control part lid plate 33 which closes an upper portion of the control part 30, and a control part bottom plate 34 which closes a lower portion of the control part 30. An air inlet 35 and an air outlet 36 are provided to both opposing side surfaces of the control part 30 for cooling the ultrasonic oscillation generating device 31 stored in the control part 30.

The ultrasonic oscillation generating device 31 is mounted on an upper surface of the control part bottom plate 34, and the ultrasonic oscillation generating device 31 plays a role of generating an electric signal having predetermined frequency when electricity is supplied to the ultrasonic oscillation generating device 31 from a power source not shown in the drawings, and a role of transmitting the electric signal to the ultrasonic oscillators 11 connected thereto thus generating the ultrasonic waves.

Legs 37 which are made of an elastic material are arranged on a lower surface side of the control part bottom plate 34 thus preventing the propagation of oscillations of the active oxygen containing water producing apparatus A generated along with the driving of the ultrasonic oscillators 11 to the surrounding.

A cooling fan 38 which is driven by a power source not shown in the drawing is provided to the water inlet 35, while a mesh plate 39 having meshes which allows the circulation of air is provided to the air outlet 36. Due to such a constitution, the ultrasonic oscillation generating device 31 is cooled by an air flow which is generated by the cooling fan 38, and air can be discharged through the mesh plate 39.

The active oxygen containing water producing apparatus A according to this embodiment having the above-mentioned constitution is driven as follows.

Firstly, when water (running water) is supplied to the water inlet 13, water is gradually filled in the inside of the reaction part 10 (inside of the reaction part outer sleeve 12), and the catalytic body 25 is immersed in water. Further, water flows out from the water outlet 27.

The first water flow which flows between the outer surface of the cell body 17 and the inner peripheral surface of the catalytic body 25 is generated in the first flow passage 50, and the second water flow which elevates along the helical plate 26 is generated in the second flow passage.

Here, with respect to the second flow passage, the second water flow is elevated while possessing a centrifugal force along an inner peripheral surface of the reaction part outer sleeve 12 and hence, to compare a water pressure in the vicinity of the inner peripheral surface of the reaction part outer sleeve 12 and a water pressure in the vicinity of an outer peripheral surface of the catalytic body 25 with each other, the water pressure in the vicinity of the outer peripheral surface of the catalytic body 25 becomes smaller.

Further, the first water flow which directly flows into the first flow passage from the water inlet 13 is also water which flows through a bypass which connects the water inlet 13 and the water outlet 27 with a short distance and hence, the first water flow exhibits a low pressure loss attributed to resistance and possesses a relatively high pressure.

Accordingly, in the vicinity of the inner and outer peripheral surfaces of the catalytic body 25, due to the difference in water pressure between the first flow passage 50 and the second flow passage 51, the third water flow which reaches the second flow passage from the first flow passage 50 after passing through the inside of the catalytic body 25 (passing through the third flow passage) is generated.

In such a state, when electricity is supplied to the ultrasonic oscillation generating device 31, ultrasonic waves are generated by the ultrasonic oscillators 11, and ultrasonic waves are applied to water (running water) in the inside of the reaction part 10 and the catalytic body 25.

Active oxygen species is generated on the surface of the catalytic body 25 to which the ultrasonic waves are applied, and active oxygen species freely float in water in the vicinity of the catalytic body 25.

Further, also with respect to active oxygen species which is generated in the inside of the catalytic body 25, active oxygen species is mixed into the second water flow through the third water flow thus producing water containing active oxygen species. Then, water containing active oxygen species is discharged from the water outlet 27 and is used in various applications.

In addition to the above-mentioned constitution, when electricity is supplied to an electricity supply part 22 of the electromagnetic wave generation device 21, electromagnetic waves are generated from tube parts 23, and electromagnetic waves are radiated to the catalytic body 25 through the cell body 17.

Due to such a constitution, active oxygen species generated on the surface of the catalytic body 25 are further increased thus producing water containing active oxygen species which contains a larger quantity of active oxygen species.

### [Another embodiment of active oxygen containing water producing apparatus]

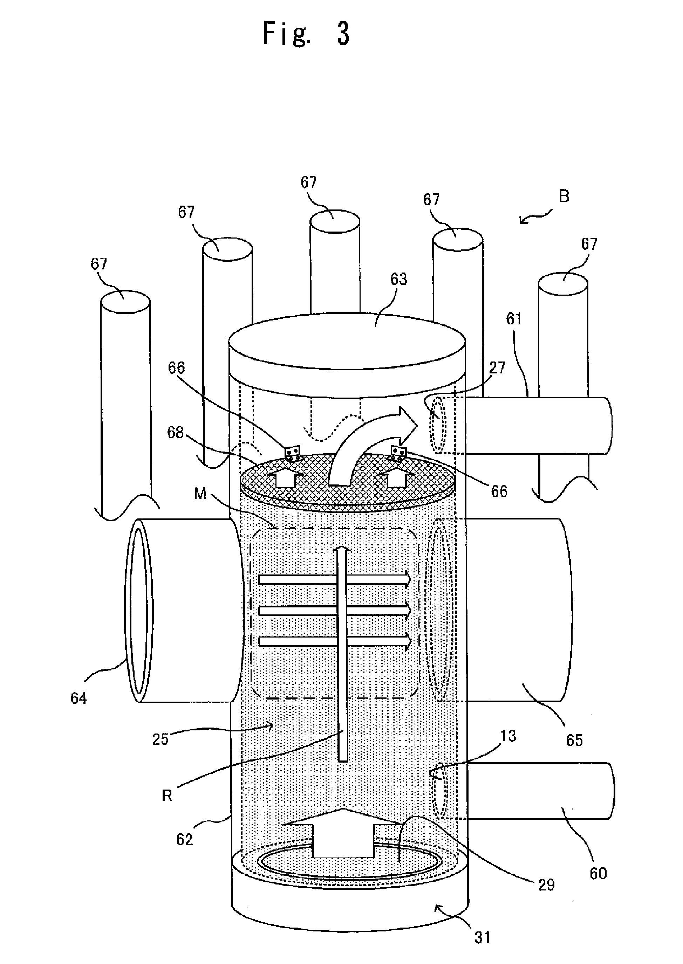

Next, the specific constitution of the active oxygen containing water producing apparatus B according to another embodiment is explained in conjunction with Fig. 3. In the explanation of the active oxygen containing water producing apparatus B hereinafter, the constitutions substantially equal to the above-mentioned constitutions are given the same symbols and the explanation of the constitutions is omitted.

The active oxygen containing water producing apparatus B of this embodiment is characterized in that a water supply pipe 60 and a water discharge pipe 61 are connected to the active oxygen containing water producing apparatus B, active oxygen species is put into water to be supplied through the water supply pipe 60, and water can be taken out from the water discharge pipe 61 whereby active oxygen containing water can be produced in line.

To be more specific, in the active oxygen containing water producing apparatus B, a hermetic vessel is formed by closing upper and lower openings of a transparent tubular body 62 made of a material which absorbs a small amount of ultraviolet rays or microwaves, that is, a transparent material which allows the transmission of ultraviolet rays and microwaves with an upper lid body 63 and an ultrasonic oscillation device 31 respectively. As the transparent material which constitutes the tubular body 62 and allows the transmission of ultraviolet rays and microwaves, for example, quartz glass, an acrylic resin and the like can be named.

Further, a water inlet 13 of the water supply pipe 60 and a water outlet 27 of the water discharge pipe 61 face a wall surface of the tubular body 62 in a communicable manner, and water which is supplied through the water supply pipe 60 by way of the water inlet 13 is turned into active oxygen containing water in the tubular body 62, and is discharged from the tubular body 62 by way of the water outlet 27.

Further, a catalytic body 25 is accommodated or filled in the tubular body 62. In the drawing, symbol 68 indicates a disc-shaped mesh body for preventing the flowout of the catalytic body 25 to the water discharge pipe 61, and the mesh body 68 is fixed to an inner wall surface of the tubular body 62 by a mounting fixing 66.

The catalytic body 25 is formed of fibers having free ends thereof vibrated by ultrasonic oscillations which are used in the above-mentioned active oxygen containing water producing apparatus A. In the inside of the catalytic body 25, pores which also allow ultraviolet rays to pass through the active oxygen containing water inwardly by 1.5cm from an inner wall surface of the tubular body 62 are formed in a communicable manner. The catalytic body 25 is excited by receiving ultraviolet rays which are irradiated from an ultraviolet ray lamp 67 which is arranged around the tubular body 62 and constitutes an ultraviolet ray irradiation part thus allowing water to contain active oxygen species therein. That is, in the active oxygen containing water producing apparatus B according to another embodiment, the ultraviolet ray lamp 67 is arranged around the periphery of the vessel such that the ultraviolet ray lamp 67 can irradiate ultraviolet rays to the catalytic body 25 whereby the substantially whole inside of the tubular body 62 forms an ultraviolet ray irradiation region.

Further, an ultrasonic wave oscillation device 31 which is arranged on one-end-side opening of the tubular body 62 and constitutes an ultrasonic wave irradiation part includes an oscillation plate 29 which irradiates ultrasonic waves into water. The oscillation plate 29 is arranged in an exposed state on an inner bottom portion of the tubular body 62 so that the oscillation plate 29 can irradiate ultrasonic waves into water which passes through the catalytic body 25 or the pores of the catalytic body 25. It is preferable to arrange the oscillation plate 29 of the ultrasonic wave oscillation device 31 at a position where a microwave irradiation range M described later falls within 10 to 15cm from the oscillation plate 29. When the oscillation plate 29 is arranged outside the range of such distance (ultrasonic wave irradiation range), although it depends on an output of the ultrasonic wave oscillation device 31, when a practically available ultrasonic wave oscillation device 31 is used, the attenuation of ultrasonic waves is remarkable thus giving rise to a possibility that ultrasonic waves cannot be radiated to the catalytic body 25 and water sufficiently.

Further, on an outer wall surface portion of the tubular body 62, a microwave supply tube 64 which extends from a wall of a microwave generation device not shown in the drawing and a microwave return tube 65 are arranged. Microwaves which are irradiated from the microwave supply tube 64 permeate a wall of the tubular body 62 and passes through the inside of the catalytic body 25 (indicated by a blanked arrow directing toward the right from the left in the drawing), and permeate the tubular body 62 and reaches the microwave return tube 65. In the drawing, a portion indicated by a broken-line frame indicates the microwave irradiation range M through which microwaves pass. The microwave supply tube 64 and the microwave return tube 65 play a role of a microwave irradiation part.

Here, with respect to a length of the microwave irradiation range M from a contact portion between the microwave supply tube 64 and the tubular body 62 to a contact portion between the microwave return tube 65 and the tubular body 62, although it may depend on an output of the microwaves or the like, it is preferable to set the length to approximately 1 to 3cm. When the length is below 1cm, a flow rate of water or active oxygen containing water which flows in the catalytic body 25 is lowered, while when the length exceeds 3cm, all microwaves are absorbed by the catalytic body 25 and water thus giving rise to a possibility that there exists the catalytic body 25 or water to which microwaves are not irradiated. Accordingly, the length exceeding 3cm is not desirable.

In the active oxygen containing water producing apparatus B having such a constitution, water supplied from the water supply pipe 60 reaches the inside of the tubular body 62 by way of the water inlet 13 and, as the whole, flows in the direction indicated by an arrow R by passing through a helical flow passage formed in the inside of the catalytic body 25 along an inner peripheral wall of the tubular body 62 or flow passages which are formed by connecting pores in the catalytic body 25, and moves toward the water outlet 27.

Here, ultrasonic waves generated by the oscillation plate 29 of the ultrasonic waves oscillation device 31 are applied to water and microwaves are applied to water from the microwave supply tube 64 so that it is possible to generate a photocatalyst similar reaction in water. That is, energy of the microwaves excites metal oxide atoms (XnOm, for example, TiO₂, Al₂O₃ or the like) on a surface of a metal oxide film thus generating free electrons and holes (photons) whereby the catalytic body 25 to which energy of ultraviolet rays and energy of microwaves are synergistically imparted can generate a large quantity of active oxygen species in water.

Then, such water flows in the inside of the tubular body 62 in the direction indicated by an arrow R and becomes active oxygen containing water which contains a large quantity of active oxygen species. Active oxygen containing water reaches a mesh body 68, and reaches the water discharge pipe 61 by way of the water outlet 27 and is discharged. Although water supplied to the tubular body 62 from the water inlet 13 passes through a complicated flow passage by turning along an inner wall surface of the tubular body 62 and by moving through the pores of the catalytic body 25, water substantially flows in the direction indicated by an arrow R toward the water outlet 27.

In this manner, this active oxygen containing water producing apparatus B also can generate active oxygen containing water by allowing water to contain active oxygen species. Further, it is possible to irradiate ultrasonic waves, microwaves and ultraviolet rays to water simultaneously and hence, it is possible to allow water to contain active oxygen species at high efficiency. In other words, at least a portion of the catalytic body 25 is arranged in a region where a microwave irradiation region to which microwaves are irradiated from a microwave irradiation part, an ultrasonic wave irradiation region to which ultrasonic waves are irradiated from an ultrasonic wave irradiation part, and an ultraviolet-ray irradiation region to which ultraviolet rays are irradiated from an ultraviolet ray irradiation part overlap with each other and hence, it is possible to allow water to contain active oxygen species efficiently.

Further, the active oxygen containing water producing apparatus B per se can be used in line so that the active oxygen containing water producing apparatus B can have a compact shape.

The catalytic body 25 in the active oxygen containing water producing apparatus B is used in a mode where the above-mentioned fibers are filled in the tubular body 62. However, the present invention is not limited to such a mode. A catalytic body which is formed of a cotton-like collective body of the above-mentioned fibers or a powdery or beads-like catalyst (for example, granular carriers covered with a catalyst by coating) may be accommodated in the tubular body 62 as the catalytic body 25.

Next, a content of active oxygen contained in water containing active oxygen species produced by the active oxygen containing water producing apparatus A is measured.

In confirming the development of various kinds of active oxygen species, the following circulating system is built as an experiment system for confirming the development of active oxygen species. That is, in the circulating system, circulating water is introduced into a precursor adding device which uses a membrane oxygenator (Synthesis M made by Solin Biomedica Japan K.K.) using a small centrifugal pump (Bio Pump made by Medtronic Japan, Inc.) thus forcibly elevating the concentration of dissolved precursor. Then, the circulated water is supplied to the active oxygen containing water producing apparatus A from the water reservoir thus producing water containing developed active oxygen. Thereafter, the water is returned to the water reservoir.

It is possible to control a flow rate, a water temperature and the concentration of oxygen dissolved in water circulated in this system to fixed values within a range of from 500ml/minutes to 20L/minute, within a range from 0°C to 43°C, and within a range from 1 to 45mg/L respectively.

Further, it is possible to eliminate influence of bubbling which is generated when oxygen is directly administered to the inside of the circuit to increase oxygen concentration. The precursor adding device is not limited to a membrane oxygenator and, provided that the dissolved gas concentration can be controlled, any means can be used as the precursor adding device without causing a problem. In an actual use, the concentration of the dissolved gas may be controlled in the following manner. That is, the gas may be mixed into the circuit by direct bubbling, and further, a size of the mixed gas bubbles may be reduced by a stirring device so as to allow the gas to be mixed as minute bubbles or, the gas may be dissolved in water as a gas in a smaller molecular state by applying ultrasonic oscillations to the small bubbles.

Further, here, oxygen concentration is exemplified as a concentration of the gas to be controlled, that is, the adjustment of dissolved oxygen concentration is exemplified as a concentration of the gas to be controlled. However, depending on a kind of the targeted active oxygen species, it is possible to control the concentration of the dissolved gas by mixing ozone or oxygen containing a large quantity of ozone (it is possible to generate highly-concentrated oxygen containing 300ppm of ozone by giving pure oxygen to a ceramic ozonizer), a nitrogen monoxide gas, a chlorine gas or the like.

The reduction of the quantity of the circulating water caused by incorporating an atomization ultrasonic oscillator in the apparatus is hardly recognized.

The following confirmation of generation of superoxide anion radicals is performed under the following condition. 10L of water is circulated at a circulation flow rate of 15L/min, oxygen is added as a precursor substance, the dissolved oxygen concentration is set to 30mg/L, two sets of atomization-use 2.4MHz ultrasonic oscillators (HM-2412, atomization capacity 250±50ml/h (water, 25°C)) or two sets of atomization-use 1.6MHz ultrasonic oscillators (HM-1630, atomization capacity 575±125ml/h (water, 25°C)) are used as the ultrasonic oscillators 11 arranged on the active oxygen containing water producing apparatus A, and a black light (EFD15BLB made by Toshiba Lighting & Technology Corporation, peak wavelength: 352nm, ultraviolet ray output: 1.8W) is used as the electromagnetic wave generation device 21.

To prevent the confusion, "water which contains a large quantity of active oxygen species and can keep the active oxygen species for a long time" is expressed as "ROS-W (Reactive Oxygen Species Water)", "water which contains a large quantity of superoxide anion radicals and can keep the superoxide anion radicals for a long time" is expressed as "SA-W (Superoxide Anion radical Water)" and "water which contains a large quantity of singlet oxygen and can keep singlet oxygen for a long time" is expressed as "SO-W (Singlet Oxygen rich Water)".

### [Confirmation of generation of superoxide anion radicals]

The measurement of the superoxide anion radicals in water is performed in the following manner. That is, using Cypridina luciferin analog derived from sea fireflies (hereinafter abbreviated as CLA) which is a chemiluminescent reagent which is peculiar to superoxide and exhibits reactivity to singlet oxygen to some extent, the chemiluminescence dependent on CLA is detected by a luminometer and is recorded.

Further, timings for collecting samples are set to immediately before the starting of reaction, 15 minutes after the start of reaction, and 30 minutes after the starting of reaction. At respective timings, 500µl of aqueous solution in the reservoir is sampled using a micropipette and is added to 500µl of 25mM phosphoric acid potassium buffer solution (pH7.0) to which CLA is preliminarily added (1:1 mixture, total: 1ml) and CLA luminescence is measured for 5 minutes after dropping the solution. The intensity of luminescence is indicated by relative luminescence unit (hereinafter referred to as rlu).

Fig. 4 shows a result of change with time of concentration of superoxide anion radicals obtained by CLA luminescence. As shown in Fig. 4, in spite of a fact that the active oxygen containing water is taken out from the reservoir, that is, the active oxygen containing water is separated from the reaction field with the catalyst and is left in the luminometer, CLA luminescence which is maintained for a long time is confirmed from the active oxygen containing water. Although CLA luminescence having a spike shape should be originally confirmed, as shown in Fig. 4, a luminescence quantity is gradually increased for 10 minutes after the measurement is started and, then, the luminescence quantity is gradually decreased.

Generally, since superoxide anion radicals are extremely reactive and unstable, superoxide anion radicals instantaneously disappears in water.

However, from this result, it is examined that the generation of superoxide anion radicals is continuously performed for a long time even outside the apparatus in water which is made to pass through the active oxygen containing water producing apparatus A, that is, in active oxygen containing water.

Further, the time is extremely long, and the reaction continues even the time exceeds 30 minutes after the detection is started.

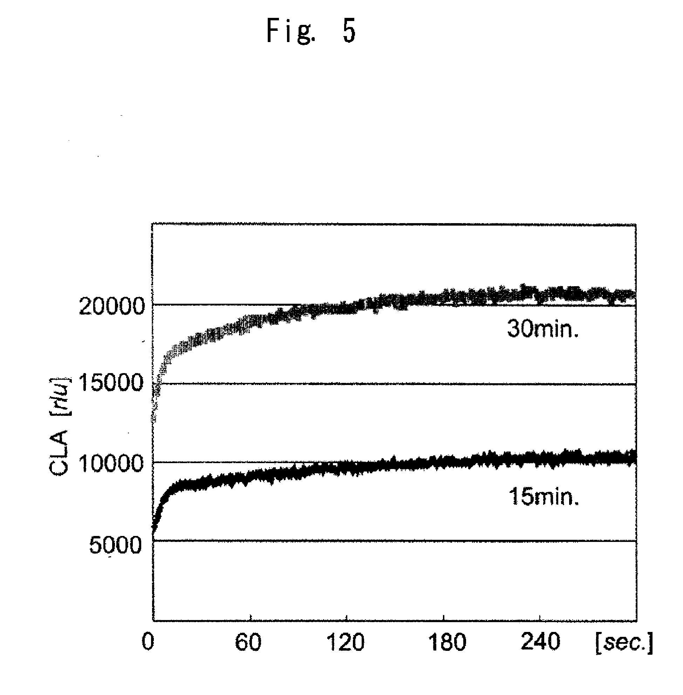

Further, as shown in Fig. 5, the longer the time during which water is circulated by the active oxygen containing water producing apparatus A, larger the quantity of the superoxide anion radicals becomes and, the generation quantity is increased with the lapse of time even after sampling the water.

Generally, it is known that, although CLA is used as a specific detection reagent of superoxide anion radicals, CLA shows reactivity to singlet oxygen to some extent.

To examine whether or not the observed chemiluminescence reflects the generation of superoxide anion radicals, Tiron which is a remover of superoxide and DABCO which is a remover of singlet oxygen are added. Due to such an examination, it is confirmed that the CLA luminescence which is observed this time reflects the generation of superoxide anion radicals in a single form.

In the same manner, the detection of hydrogen peroxide is also performed using luminol as chemiluminescence substrate, using horseradish peroxidase as a detection catalyst under the neutral pH condition.

As a result, luminol luminescence is detected, and it is clarified that hydrogen peroxide of extremely low concentration of 1 to 20nmol/L exists in water which has passed through the active oxygen containing water producing apparatus A.

However, the generation quantity of hydrogen peroxide changes for every measurement time and hence, it is suggested that hydrogen peroxide involves in a reaction process of active oxygen water species. However, since the hydrogen peroxide contained in water exhibits the extremely low concentration, the hydrogen peroxide cannot generate the redox reaction to a living body attributed to extracellular stimulation.

Further, since it is considered that ozone involves in the reaction, ozone in air in the vicinity of reservoir is detected by a gas detecting tube. However, no ozone is detected at all. The presence and non-presence of ozone are confirmed using a calorimetric method by indigo carmine in circulating water. However, ozone is not detected at all and hence, it is confirmed that generation of ozone is extremely low.

Further, KO₂ solution obtained by dissolving KO₂ (potassium superoxide) in an organic solvent is added to CLA solution thus preparing a calibration curve of superoxide anion by chemiluminescence dependent on CLA. Generally, a method which dissolves KO₂ in crown-ether-based organic solution and uses the solution for the determination of superoxide is used. However, to allow the reaction with CLA in aqueous solution in a non-hydrophobic environment, dimethylsulfoxide (DMSO) is used as organic solvent. Using this method, CLA luminescence by adding KO₂ solution is induced whereby the calibration curve of superoxide anion radicals is prepared.

As a result, water containing active oxygen generated by the active oxygen containing water producing apparatus A is characterized in that the water can be produced as water containing a large quantity of SA-W, that is, superoxide anion radicals and allows the SA-W to be held and to function for a long time and contains 200µ mol/L of superoxide radicals at maximum.

### [Confirmation of generation of singlet oxygen]

Next, in the substantially same circulation system as the system in which the above-mentioned confirmation of generation of superoxide anion radical is performed, the substantially same measurement as the above-mentioned measurement is performed using 10L of ozone water having the initial concentration of 2.5ppm as circulating water. To produce ozone water containing a large quantity of ozone as precursor substance for generating active oxygen species, Quick Ozone 10 made by Ai Electronic Industry Co., Ltd which produces ozone water using an ionization method is used. The concentration of dissolved ozone is measured using a calorimetric method by indigo carmine.

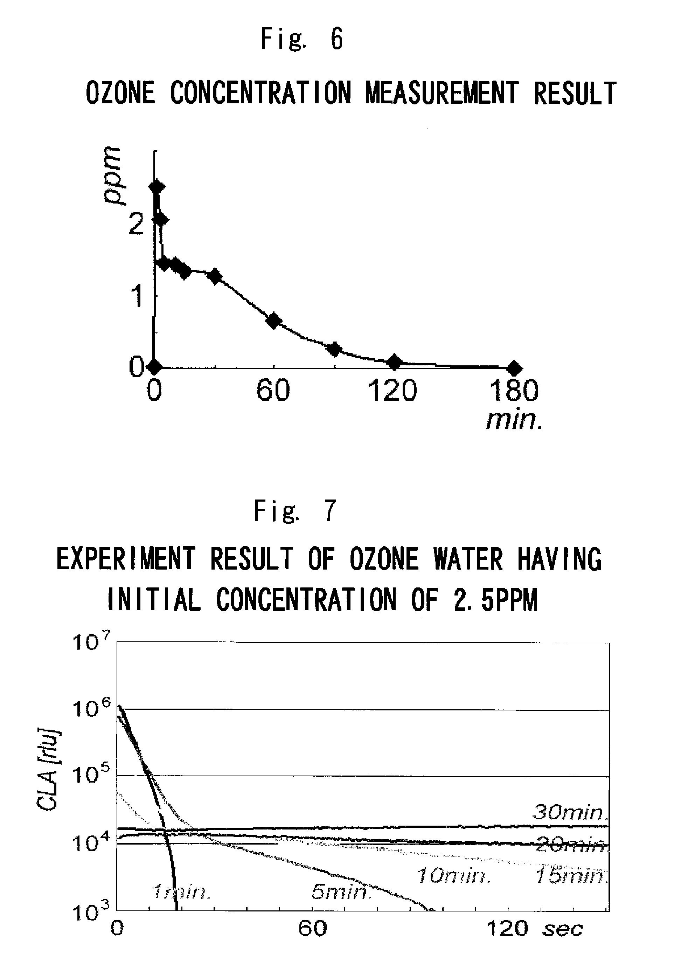

As shown in Fig. 6, water containing ozone having the initial concentration of 2.5ppm as a precursor substance maintains the concentration of approximately 1.5ppm for approximately 30 minutes when the ozone water does not react with catalytic body. As a prior art, it is known that ozone is decomposed by ultraviolet rays at a level of a bactericidal lamp. However, UV-A (365nm) which induces a photocatalytic reaction does not prompt the decomposition of ozone so that ozone at high concentration is maintained.

The result of experiment using ozone water having initial concentration of 2.5ppm is shown in Fig. 7. Under the presence of catalytic body, the explosive chemiluminescence of CLA luminescence which exceeds 1000000 [rlu] at a maximum value is generated for five minutes after applying ultraviolet rays (UV-A) radiation and ultrasonic oscillations, and ozone is decomposed to a level of ozone concentration at 0.2ppm, that is, to a level where ozone activity is hardly obtained when 3 minutes elapse after the reaction is started.

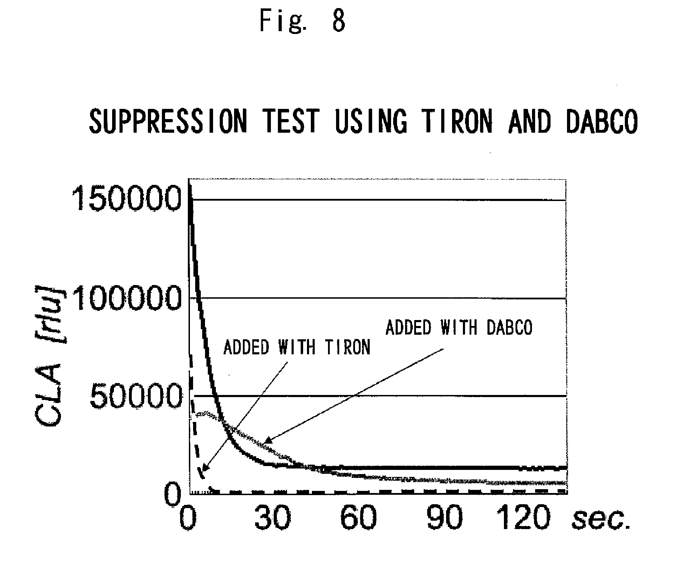

Fig. 8 shows a result of an inhibiting experiment performed by adding Tiron which is a superoxide removing agent and DABCO which is a singlet oxygen remover to a sample when three minutes elapse after the reaction starts.

From Fig. 8, it is found that most of CLA luminescence having a spike shape is derived from the generation of singlet oxygen. Since there is no marker corresponding to the scale of singlet oxygen, it is found that active oxygen species at high concentration equivalent to 1.7mM/L in terms of superoxide anion radicals can be generated. When the water to be treated is ozone water, it is possible to constantly generate water containing a large quantity of SO-W, that is, singlet oxygen and maintaining activity of oxygen species for a long time.

Next, the SAR expression effect which provides the expression of PR genes for plants is observed using these SA-W and SO-W.

The expression of PR gene group is confirmed using a PCR method which is performed after RNA extracted from a plant specimen is transformed into cDNA by reverse transcriptase (RT) (RT-PCR method).

In the pathogen-resistance genes, although the same gene is expressed depending on plant species, when the plant species differ from each other, the sequences of genes differ and there may be a case where the plant species is given different names. In tobacco (Nicotiana tabacum L.), the most representative gene of a salicylic acid responsive PR gene group which becomes an index of SAR is a PR1a gene and the PR-1 gene is also identified in the same manner with tomato (Solanum lycopersicum) as the most representative gene of a salicylic acid responsive PR gene group.

A plant body or cells of a Bel-W3 system which is a tobacco of ozone sensitive system (possessing property of low resistance to active oxygen) and a plant body or cells of a Bel-B system which is a tobacco of ozone resistance system (possessing property of high resistance to active oxygen) have the common pathogen-resistance gene PR1a. Since the sequence of the PR1 gene of tomato differs from the sequence of the PR1 gene of tobacco, the detection of the expression by RT-PCR requires a primer having the DNA sequence different from the DNA sequence of tobacco.

Primers (5'-GTAATATCCCACTCTTGCCGTGCC-3' and 5'-CGTGAAATGGACGTAGGTCG-3') which is used for amplifying a region of 423bp in the tobacco PR1a pathogen-resistance gene which is used for confirming the expression of the pathogen-resistance gene are designed in accordance with a base sequence of tobacco PR1a cDNA. In the same manner, primers (5'-GGTTAAGGCTGGATTTGCTG-3' and 5'-CCACCACCTTGATCTTCATG-3') are designed in accordance with a base sequence of tobacco Actin cDNA and are used in a control experiment as genes whose expression levels are not influenced by a stimulus. An annealing temperature (Tm) and PCR cycles at the time of performing PCR are Tm: 58 and Cycle: 30 with respect to tobacco PR1a and are Tm: 60 and Cycle: 30 with respect to tobacco Actin.

Further, primers (5'-CTTCTCATGGTATTAGCC-3' and 5'-CCACCATCCGTTGTTGC-3') which amplify 393bp of tomato PR-1 gene are designed in accordance with a base sequence of cDNA of a tomato PR-1 gene and, in the same manner, primers (5'CACACTGTCCCTATTTACGA-3' and 5'-GTAATAACTTGTCCATCAGG-3') are designed in accordance with a base sequence of tomato Actin cDNA and are used as controls. An annealing temperature (Tm) and PCR cycles for performing gene coupling at the time of PCR are Tm: 54.8 and Cycle: 30 with respect to tomato PR-1 and are Tm: 58 and Cycle: 30 with respect to tomato Actin.

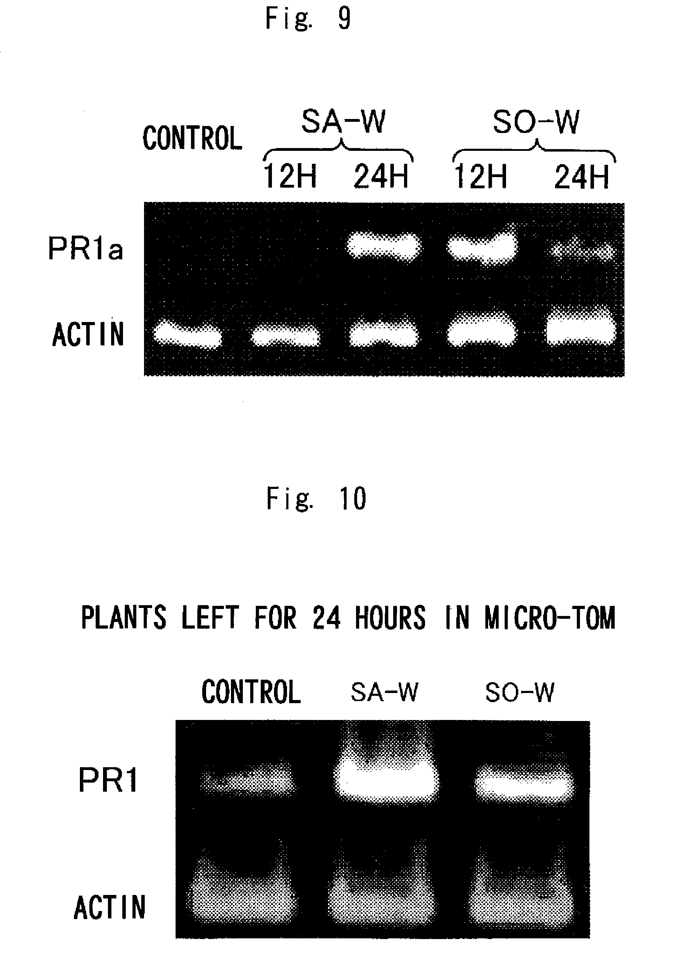

Firstly, Fig. 9 shows an agarose electrophoresis photograph which verifies the expression of tobacco PR1a genes. Data shown in Fig. 9 are obtained in the following manner. Leaf discs (diameter 10mm, 25 pieces of leaf discs for every 1 treatment zone) which are prepared from a plant body of Bel-W3 system having high sensitivity to an oxidation stress are gently placed in a plastic culture plate having a diameter of 6cm, and 30ml of SO-W (sampled from 10L of water which is circulated for 30 minutes or more under conditions where ultraviolet rays and ultrasonic waves are optimized under the presence of a catalyst (concentration of superoxide anion radicals: 200µmol/L) using a pipet or 30ml of SO-W (sampled at a point of time that a reaction occurs for 3 minutes under conditions where ultraviolet rays and ultrasonic waves are optimized under the presence of a catalyst in the inside of a apparatus filled with 10L of ozone water which contains ozone with initial concentration of 2.5ppm as an active oxygen species precursor) using a pipet is added to the leaf discs. Then, the leaf discs are left for 12 hours or 24 hours at a room temperature (under light shielding condition). A specimen in which the expression of gene is promoted is frozen and broken and, thereafter, RNA is extracted and an analysis of the expression of target genes is performed in accordance with RT-PCR.

Fig. 9 shows the result of (1) a control experiment zone (immediately after preparing leaf discs, described as Control in the drawing), (2) specimen after leaving for 12 hours after addition of SA-W (described as SA-W 12H in the drawing), (3) specimen after a lapse of 24 hours after addition of SA-W (described as SA-W 24H in the drawing), (4) a specimen after a lapse of 12 hours after addition of SO-W(described as SO-W 12H in the drawing), and (5) a specimen after a lapse of 24 hours after addition of SO-W (described as SO-W 24H in the drawing). As can be understood from the result, compared to an Actin gene whose expression level is not changed by stimulus, the expression level of the PR1a gene is remarkably elevated, and this indicates that the expression of the PR1a gene is induced by SA-W and SO-W. Although data immediately after preparation of leaf discs is shown as the control experiment in Fig. 9, it is also confirmed that the addition of running water does not induce the expression of the PR1a gene after a lapse of 12 hours and after a lapse of 24 hours.

Further, what must be noted here is that SO-W can induce the expression of pathogen-resistance gene earlier than SA-W.

It is considered that this implies that singlet oxygen induces a stronger redox reaction and the expression of pathogen-resistance gene is induced by way of the intracellular stimulus.

On the other hand, also when SA-W and SO-W are added to the leaf discs prepared from tobacco plant body of a Bel-B system having low sensitivity to an oxidation stress under the above-mentioned conditions, it is confirmed that the expression of the PR-1a genes is induced although the expression level is lower than the expression level of PR-1a gene of tobacco plant body of a Bel-W3 system. It is thought that the difference in degree of induced expression between both tobacco of Bel-B system and tobacco of Bel-W3 system is attributed to the difference in sensitivity to oxidation stress originally existing between tobaccos of both systems. The similar difference is confirmed in an expression inducing experiment of PR-1a in a case where SA-W is directly added to tobacco plant bodies of both systems. That is, in the plant body of Bel-W3 system, the expression of PR1a is remarkably induced even when SA-W treatment is performed for a short time, while the plant body of Bel-B system requires treatment of a stronger level.

This result implies the acquisition of a remarkable advantageous effect that the expression of PR-1a gene is induced by stimulating only once and by direct addition to the leaf. This result also implies that, to surely generate stimulation, by applying stimulus for a plurality of times and by applying stimulus continuously, the PR-1a gene can be effectively expressed so that the SAR induction of the plant body as a whole is secured.

Further, it is safe to say that pathogen-resistance can be effectively imparted to the plant by changing the degrees of treatments by SA-W and SO-W (concentration, treatment time, timing of treatment and the like) while taking the difference in sensitivity to an oxidation stress which a plant original possesses.

On the other hand, the expression induction effect of the PR1a gene of tobacco due to SA-W and SO-W is also confirmed by a cell-level experiment. That is, the expression induction effect is confirmed in an experiment where SA-W (superoxide anion radical concentration 200µmol/L) and SO-W are added to suspension cultured cells of Bel-W3 cultured in MS liquid culture medium (cells in a logarithmic growth phase of the fifth day of culture at 23°C) (RNA being extracted after a lapse of 1 to 12 hours and being verified in an RT-PCR experiment in the same manner as the above-mentioned experiment).

The above-mentioned result shows that, by bringing active oxygen containing water produced in this embodiment into contact with a plant body surface, a leaf disc surface and a cell surface of tobacco, the expression of tobacco PR1a gene can be induced by way of an oxidation stress response reaction. In this manner, since similar results are obtained at a plant body level, at a tissue level and at a cell level, it is proved that, as an advantageous effect of active oxygen water, a cell biological reaction which leads to the induction of the expression of PR gene is systemically confirmed, and this advantageous effect clearly shows the characteristic of systemic acquired resistance.

That is, it is suggested that pathogen-resistance gene of tobacco is expressed using active oxygen containing water thus preventing tobacco diseases.

Next, Fig. 10 shows an agarose electrophoresis photograph which verifies the induction of the expression of PR-1 genes in a case where SA-W and SO-W are added to a plant body of small-sized tomato species (micro tom, expressed as Micro-Tom in the drawing) which is used as a model material of molecular genetics. In this experiment, to reproduce the similar condition as hydroponic culture, plant bodies which are grown as potted plants for approximately a month after germination are washed in running water, are removed from their root systems, are gently placed in 300ml beakers, are soaked in approximately 100ml of running water, SA-W (concentration of superoxide anion radicals: 200µmol/L), SO-W so as to allow the plant bodies to absorb these waters and are left for 24 hours. Then, the plant bodies are frozen using liquid nitrogen and are broken and, thereafter, RNA is extracted from the broken plant body and an analysis of the expression of PR-1 genes is performed in accordance with RT-PCR. As a result, as shown in Fig. 10, it is found that the expression of the PR-1 genes is remarkably increased by the SO-W treatment and the SA-W treatment.

Further, with respect to a specimen after the lapse of 24 hours, data that SA-W brings about the higher gene expression induction effect than SO-W is obtained. However, as clearly shown by data of tobacco (Bel-W3) (Fig. 9), there is a tendency that SA-W and SO-W assume the maximum effect at different timings and hence, SA-W and SO-W can be used depending on a use purpose.

The above-mentioned result shows that, by bringing active oxygen containing water produced in this embodiment into contact with a plant body surface of tomato (Micro-Tom), in both cases of SA-W and SO-W, the expression of tomato PR-1 gene can be induced.

That is, it is suggested that pathogen-resistance gene of tomato is expressed using active oxygen containing water thus preventing tomato diseases.

Tobacco and tomato which exhibit the above-mentioned results of experiment are plants which differ in genus in systematic botany. Tobacco is a plant species which represents a plant whose main product is leaves and tomato is a plant species which represents a plant whose main product is fruits respectively. It is needless to say that these plants are important in agriculture, but these plants are also academically important as agricultural and plant physiological model plant materials respectively. Accordingly, from results shown in Fig. 9 and Fig. 10, it is thought that active oxygen containing water can induce the pathogen-resistance gene of any plants which differ in species or in genus and allow the pathogen-resistance gene to express gene product thereof thus preventing diseases of the plant.

Here, in the above-mentioned induction of the pathogen-resistance gene, SA-W and SO-W are used individually from each other. However, active oxygen containing water which use SA-W and SO-W in combination may be prepared or produced and is used for a specific plant.

That is, pathogen-resistance genes of the plant may be induced such that active oxygen containing water is prepared by mixing SO-W which contains singlet oxygen and SA-W which contains superoxide anion radicals exhibiting lower reactivity than singlet oxygen and is capable of keeping a function thereof for a longer time with each other or by directly producing water which contains singlet oxygen and superoxide anion radicals. Then, the water containing both active oxygen species is brought into contact with the plant.

In this case, singlet oxygen is used as the first active oxygen, and superoxide anion radicals are used as the second active oxygen. Here, the first active oxygen and the second active oxygen are not limited to singlet oxygen and superoxide anion radicals, and are properly selected from a group consisting of superoxide anion radicals, hydroxy radicals, singlet oxygen and oxygen-containing organic radical species. Out of the first active oxygen and the second active oxygen, one active oxygen exhibits lower reactivity and is capable of keeping a function thereof for a longer time compared to the other active oxygen.

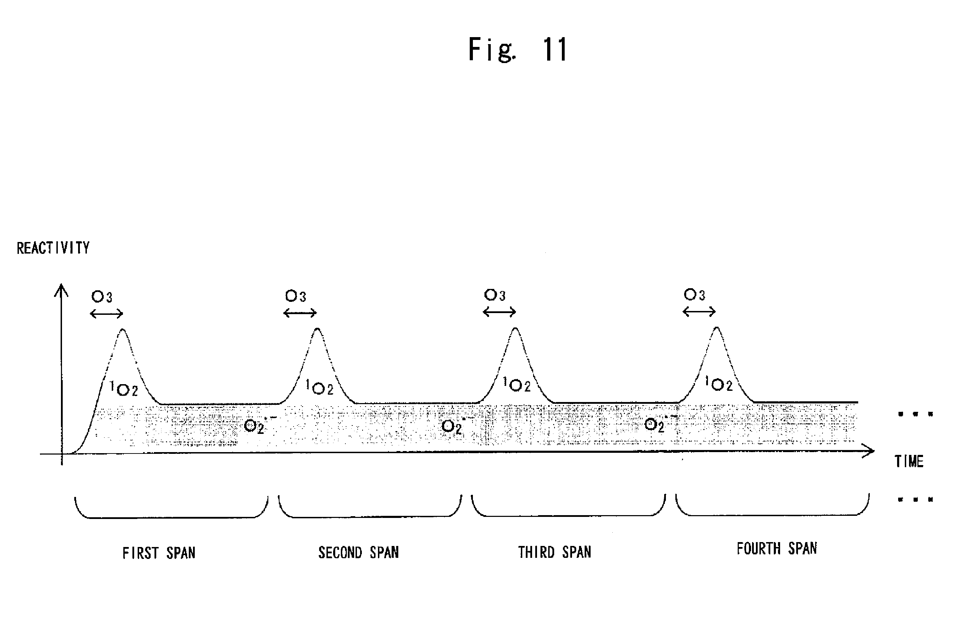

To be more specific, in such active oxygen containing water, as shown in Fig. 11, it is desirable that singlet oxygen and superoxide anion radical are present at a rate that a reaction generated by singlet oxygen has a spike-like projecting reactive balance compared to a reaction generated by superoxide anion radicals. Fig. 11 is a graph showing a change with time of reactivity in active oxygen containing water which contains singlet oxygen and superoxide anion radicals, wherein reactivity (CLA emission light quantity) is taken on an axis of ordinates, and time is taken on an axis of abscissas.

To focus on an amount of singlet oxygen and superoxide anion radicals corresponding to 1 span also shown in Fig. 11, superoxide anion radicals exhibit a prolonged lifetime and low reactivity compared to singlet oxygen and hence, superoxide anion radicals act slowly and form a baseline in the graph.

On the other hand, singlet oxygen exhibits a short lifetime and high reactivity and hence, singlet oxygen acts quickly and forms a spike-like peak in the graph.

By continuously using such active oxygen containing water for plant over several spans, the plant is allowed to continuously express pathogen-resistance genes which the plant possesses and hence, it is also possible to provide plant which is always strong against diseases.

In addition, by always applying a load to a plant by superoxide anion radicals which act slowly and, at the same time, by applying a larger load to the plant in a short time by singlet oxygen in pulse-like manner so that it is possible to further enhance the efficiency of the expression of pathogen-resistance genes compared to a case where SA-W or SO-W is used in a single form.

Further, singlet oxygen which possesses high reactivity and acts quickly plays a role of sterilizing microorganisms such as bacteria or virus which infect the plant. On the other hand, superoxide anion radicals which possess low reactivity and act slowly play a role of gradually expressing pathogen-resistance genes in the plant in a state where the infection of microorganisms is suppressed by singlet oxygen.

Further, the present invention is also characterized in that superoxide anion radicals and singlet oxygen which are contained in active oxygen containing water have extremely short lifetime compared to hydrogen peroxide or an aromatic compound such as a salicylic acid which is conventionally used for expressing pathogen-resistance genes of plant. Accordingly, superoxide anion radicals and singlet oxygen do not accumulate in soils or plants and hence, there exists no possibility that superoxide anion radicals and singlet oxygen brings about soil pollution and health damages to human. The same goes for a case where SA-W or SO-W is used in a single form.

The conspicuous expression of PR-1 is also confirmed in a Micro-Tom plant body which is continuously held in 10L of SA-W (concentration of superoxide anion radicals: 200µmol/L) for 24 hours in a state where the Micro-Tom plant body is fixed to a floating body made of foamed polystyrene, wherein SA-W is circulated under a condition where ultraviolet rays and ultrasonic waves are optimized under the presence of a catalytic body. Although the Micro-Tom plant body is continuously brought into contact with reacting SA-W which continues a reaction for 24 hours, damage to the plant body is not confirmed at all. This implies that when SA-W is actually used for hydroponic culture of tomatoes, the method which continuously processes SA-W can efficiently impart pathogen resistance to tomatoes.

It is confirmed that damage to plant per se is small even in a cell level experiment. That is, even after a lapse of 12 hours from a point of time where SA-W (concentration of superoxide anion radicals: 200µmol/L) and SO-W are added to suspension cultured cells derived from tobacco (Bel-W3, Bel-B) and suspension cultured cells derived from Micro-Tom, the cell death is not induced at all. On the other hand, in an experiment zone where ozone water is added to suspension cultured cells derived from tobacco (Bel-W3, Bel-B) and suspension cultured cells derived from Micro-Tom, the cell death is induced. This implies that SA-W and SO-W have succeeded in imparting a moderate oxidation stress to the cells of the plant while exhibiting low toxicity to the plant compared to ozone water. To apply pathogen resistance to the plant through the expression of SRA, it is important to impart an oxidation stress to the plant to an extent which is suitable for the treatment of crops.

As has been explained heretofore, according to the method of preventing diseases in plants of this embodiment, the method can prevent plant diseases in such a manner that active oxygen containing water containing active oxygen species and is capable of keeping a function of the active oxygen species for a long time is brought into contact with plants so that pathogen-resistance genes which the plants possess are induced.

Accordingly, as has been explained in conjunction with Fig. 9 and Fig. 10, it is possible to induce pathogen-resistance genes which the plants possess irrespective of genus and kind of plants.

Further, the above-mentioned experiment is performed such that 10L of running water is circulated at a circulation flow rate of 15L/min, oxygen is added as a precursor substance, the dissolved oxygen concentration is set to 30mg/L, only one set of atomization-use 2.4MHz ultrasonic oscillator (HM-2412, atomization capacity 250±50ml/h (water, 25°C)) is used as the ultrasonic oscillator 11 arranged on the active oxygen containing water producing apparatus A, and a black light (EFD15BLB made by Toshiba Lighting & Technology Corporation, peak wavelength: 352nm, ultraviolet ray output: 1.8W) is used as the electromagnetic wave generation device 21.

The produced SA-W exhibits superoxide anion radicals concentration of 50µmol/L so that the induction of the above-mentioned pathogen-resistance genes is not achieved. The result of the study of a defective gene strain using Arabitopsis thaliana as an experimental plant reveals that superoxide anion radicals has a property that a calcium channel is not released and, to the contrary, is closed with superoxide anion radicals of low concentration. The superoxide anion radicals concentration of 50 µmol/L is considered as a lower limit of reaction induction.

Finally, the above-mentioned respective embodiments explained heretofore are provided merely as examples, and the present invention is not limited to the above-mentioned embodiments. Accordingly, it is needless to say that various modifications are conceivable depending on design or the like without departing from the technical concept of the present invention even when the modifications differ from the above-mentioned embodiments.

### Explanation of symbols

A: active oxygen containing water producing apparatus
B: active oxygen containing water producing apparatus
10: reaction part
11: ultrasonic oscillator
13: water inlet
17: cell body
21: electromagnetic wave generation device
25: catalytic body
26: helical plate
27: water outlet
29: oscillation plate
31: ultrasonic oscillation generating device
50: first flow passage
51: second flow passage
52: third flow passage
61: helical flow passage
62: blow-off space

## Claims

1. Active oxygen containing water containing first active oxygen species and second active oxygen species which are selected from a group consisting of superoxide anion radicals, hydroxy radicals, singlet oxygen and oxygen-containing organic radical species and differ from each other in behavior with time, and one of the first active oxygen species and the second active oxygen species exhibits lower reactivity and is capable of keeping a function thereof for a longer time compared to the other active oxygen species so that oxidizing and reducing power which the active oxygen species possess is made to reach the inside of the plant cells from the outside of the plant cells and brings about a redox reaction in the inside of the plant cells thus inducing the expression of pathogen-resistance genes in the plant cells.

2. A method of preventing diseases in plants in which plant diseases are prevented by bringing active oxygen containing water containing active oxygen species and is capable of keeping a function of the active oxygen species for a long period into contact with plants so that the expression of pathogen-resistance genes which the plants possess is induced.

3. The method of preventing diseases in plants according to claim 2, wherein the active oxygen containing water contains at least one active oxygen which is selected from a group consisting of superoxide anion radicals, hydroxy radicals, singlet oxygen and oxygen-containing organic radical species so that oxidizing and reducing power which the active oxygen species possess is made to reach the inside of the plant cells from the outside of the plant cells by means of a plant contact means, and the expression of pathogen-resistance genes is induced due to a redox reaction which is brought about in the inside of the plant cells by the absorbed water whereby the plant diseases are prevented.

4. The method of preventing diseases in plants according to claim 2, wherein the active oxygen containing water contains first active oxygen species and second active oxygen species which are selected from a group consisting of superoxide anion radicals, hydroxy radicals, singlet oxygen and oxygen-containing organic radical species and differ from each other in behavior with time, and one of the first active oxygen species and the second active oxygen species exhibits lower reactivity and is capable of keeping a function thereof for a longer time compared to the other active oxygen species, and
in inducing the pathogen-resistance genes, the first active oxygen species which is capable of directly acting on pathogenic bacteria is made fast-acting and, subsequently, the second active oxygen species which is capable of inducing the expression of the pathogen-resistance in the plant is made slow-acting thus preventing diseases in plants.

5. The method of preventing diseases in plants according to any one of claims 2 to 4, wherein the active oxygen species is produced by applying at least one of ultraviolet rays, ultrasonic oscillations, a visible light, microwaves to a catalytic body immersed into water.

6. The method of preventing diseases in plants according to claim 5, wherein the water is water in which at least one of an oxygen gas, an ozone gas, a chloride gas, a nitrogen monoxide gas and an ammonium gas which constitutes a precursor of the active oxygen species is dissolved.

7. The method of preventing diseases in plants according to claim 5 or 6, wherein the catalytic body contains at least one selected from a group of metals constituted of metal oxide ions or metal hydroxide ions of titania (TiO₂), alumina(Al₂O₃), anodized aluminum, magnesium oxide, magnesium hydroxide, magnetite (Fe₃O₄), zinc oxide, tungsten oxide, barium titanate, strontium titanate, sodium titanate, zirconium dioxide, tungsten oxide, a tungsten hydroxide compound, α-Fe₂O₃, cadmium sulfide, zinc sulfide, platinum, copper, palladium.

8. The method of preventing diseases in plants according to any one of claims 5 to 7, wherein the catalytic body has a powdery form, a granular form or a fibrous form.

9. The method of preventing diseases in plants according to any one of claims 2 to 7, wherein the active oxygen containing water is produced by an active oxygen containing water producing apparatus in which an ultraviolet-ray light source is arranged in water and a periphery of the ultraviolet-ray light source is surrounded by a photocatalytic body formed of a fibrous body with a fixed gap held between the ultraviolet-ray light source and the photocatalytic body whereby a water flow is formed of a first water flow which ascends and descends by passing the gap defined between the ultraviolet-ray light source and the photocatalytic body, a second water flow which ascends and descends by spirally passing on an outer periphery of the photocatalytic body, and a third water flow which flows into or flows out from the texture of the photocatalytic body.

10. An active oxygen containing water producing apparatus used in the method of preventing diseases in plants according to any one of claims 2 to 9, the apparatus comprising:
an ultraviolet-ray light source which is arranged in a vessel provided with a water supply port and an active oxygen containing water takeout port; and
a photocatalytic body which is formed of a fibrous body and surrounds a periphery of the ultraviolet-ray light source with a predetermined gap held between the photocatalytic body and the ultraviolet-ray light source, wherein
a water flow supplied to the inside of the vessels is formed of a first water flow which ascends and descends by passing the gap defined between the ultraviolet-ray light source and the photocatalytic body, a second water flow which ascends and descends by spirally passing on an outer periphery of the photocatalytic body, and a third water flow which flows into or flows out from the texture of the photocatalytic body.

11. An active oxygen containing water producing apparatus used in the method of preventing diseases in plants according to any one of claims 2 to 9, the apparatus comprising:
a vessel which is made of a material which allows the transmission of ultraviolet rays therethrough, is provided with a water supply port and a water discharge port and houses a catalytic body in the inside thereof;
an ultrasonic irradiation part which irradiates ultrasonic waves in the flow direction of water which flows into the inside of the vessel from the water supply port; and
an ultraviolet-ray irradiation part which is arranged on the periphery of the vessel and irradiates ultraviolet rays to the catalytic body from the outside of the vessel, wherein
at least a portion of the catalytic body is arranged in an area where an irradiation area to which ultrasonic waves are irradiated from the ultrasonic irradiation part and an irradiation area to which ultraviolet rays are irradiated from the ultraviolet-ray irradiation part overlap with each other.

12. The active oxygen containing water producing apparatus according to claim 11, further comprising a microwave irradiation part which, when water which flows into the vessel from the water supply port flows toward the water discharge port, irradiates microwaves in the direction orthogonal to the direction along which the water flows in the inside of the vessel, wherein
at least a portion of the catalytic body is arranged in an area where an irradiation area to which microwaves are irradiated from the microwave irradiation part, an irradiation area to which ultrasonic waves are irradiated from the ultrasonic irradiation part and an irradiation area to which ultraviolet rays are irradiated from the ultraviolet-ray irradiation part overlap with each other.
